# EUROPEAN PATENT APPLICATION

(11) **EP 4 159 235 A1**
(43) Date of publication of application: **05.04.2023**
(21) Application number: 21811909.7
(22) Date of filing: 24.05.2021
(51) Int. Cl.: A61K 39/395, A61P 29/00, A61P 37/06, A61P 37/08, C07K 16/28

(54) **PD-1 AGONIST-CONTAINING PHARMACEUTICAL COMPOSITION FOR TREATING OR PREVENTING TH2-MEDIATED DISEASE**

(30) Priority: 25.05.2020 JP 2020090526
(71) Applicant: Foundation for Biomedical Research and Innovation at Kobe, Kobe-shi, Hyogo 650-0047 (JP); National Institutes of Biomedical Innovation, Health and Nutrition, Ibaraki-shi, Osaka 567-0085 (JP); Meiji Seika Pharma Co., Ltd., Tokyo 104-8002 (JP)
(72) Inventor: HONJO,Tasuku, Kobe-shi, Hyogo 650-0047 (JP); OHTA,Akio, Kobe-shi, Hyogo 650-0047 (JP); TAJIMA,Masaki, Kobe-shi, Hyogo 650-0047 (JP); KAMADA, Haruhiko, Ibaraki-shi, Osaka 567-0085 (JP); NAGATA, Satoshi, Ibaraki-shi, Osaka 567-0085 (JP); SUZUKI, Kensuke, Yokohama-shi, Kanagawa 222-8567 (JP); OSHIRO, Yuya, Yokohama-shi, Kanagawa 222-8567 (JP); TOKUMARU, Yosuke, Yokohama-shi, Kanagawa 222-8567 (JP)
(74) Representative: Hoffmann Eitle
(86) International application number: PCT/JP2021/019676
(87) International publication number: WO 2021/241523

(57) **Abstract**

A novel use of agonist to PD-1 is developed. Provided is a pharmaceutical composition for treating or preventing Th2-mediated diseases, the composition comprising an effective amount of a PD-1 agonist.

## Description

### TECHNICAL FIELD

The present invention relates to a PD-1 agonist-comprising pharmaceutical composition for treating or preventing Th2-mediated diseases.

### BACKGROUND ART

The immune response has an aspect that its inappropriate regulation could lead to diseases. An insufficient immune response to pathogens such as bacteria and viruses that have invaded the organism results in infectious diseases. Conversely, autoimmune diseases develop when adverse immune responses to self-tissues occur. To prevent from falling into such pathological conditions and to utilize the system effectively, the immune system is equipped with both mechanisms activating the function of immune cells to promote immune response and mechanisms suppressing immune cells to reduce immune response. Disruption of these endogenous regulatory mechanisms is considered to contribute to diseases resulting from insufficient or excessive immune response.

The best example demonstrating the importance of endogenous immunoregulation can be found in cancer immunotherapy that has rapidly become a reality in recent years. The molecules such as CTLA-4 and PD-1 appearing in this novel therapy, which is clearly distinct from conventional "immunotherapy", are a type of endogenous immunosuppressive mechanisms also called immune checkpoints, and have a particularly remarkable impact among the same type of immunosuppressive mechanisms. The pathology of cancer can be regarded as a condition where cancer cells that should have been eliminated have proliferated because of insufficient immune response thereto. The cause of this insufficient immune response is related to the fact that the inside of cancer tissues is filled with various types of immunosuppressive mechanisms. This means that cancer tissues evade attacks from immune cells by actively using the immunosuppressive mechanisms intrinsic to the organism. The blockade of CTLA-4 or PD-1, representative immunosuppressive mechanisms, reverses the inhibition of anti-tumor immunity, and the resulting immune activation brings about actual therapeutic effects. The fact shows that the above-mentioned endogenous immunosuppressive mechanisms have highly significant effects and can be important targets for the treatment of diseases by correcting the imbalance in immune response.

The critical importance of endogenous immunosuppressive mechanisms is also shown by the fact that the absence of one of these mechanisms may cause much exaggerated inflammatory responses. For example, PD-1-deficient mice have been shown to spontaneously develop various inflammatory diseases (Non-Patent Document No. 1: Okazaki et al. Nat. Immunol., 2013). Inflammatory adverse response resulting from excessive immune response is observed at a certain proportion of cancer patients undergoing immunotherapy with PD-1 inhibitors (Non-Patent Document No. 2: Young et al. Cancer Immunol. Res., 2018). These facts indicate that the immunosuppressive mechanisms that are actively utilized in cancer tissues are essentially physiological feedback mechanisms to protect healthy tissues in the body from excessive inflammatory responses. Indeed, the clinical efficacy of pharmaceutical drugs such as anti-PD-1 antibody in cancer therapy suggests that it is possible to modulate the intensity of immune response by regulating the function of PD-1.

### PRIOR ART LITERATURE

### Non-Patent Documents

Non-Patent Document No. 1: Okazaki T, Chikuma S, Iwai Y, Fagarasan S, Honjo T. A rheostat for immune responses: the unique properties of PD-1 and their advantages for clinical application. Nat. Immunol. 14:1212-1218 (2013).
Non-Patent Document No 2: Young A, Quandt Z, Bluestone JA. The Balancing Act between Cancer Immunity and Autoimmunity in Response to Immunotherapy. Cancer Immunol. Res. 6:1445-1452 (2018).

### DISCLOSURE OF THE INVENTION

### PROBLEM FOR SOLUTION BY THE INVENTION

As shown in the above-described background, drugs that inhibit immunosuppressive mechanisms were used in cancer therapy where the enhancement of immune response was desirable. However, to suppress excessive immune response in inflammatory diseases, the treatment demands an opposite approach that actively stimulates the immunosuppressive mechanisms. PD-1 is expressed on activated T cells, and upon antigen recognition, its interaction with ligand molecule such as PD-L1 or PD-L2 on the surface of other cells interferes with the activating signaling in T cells. Since PD-1 itself can initiate the immunosuppressive signaling, artificial stimulation of PD-1 might lead to the treatment of inflammatory diseases. While anti-PD-1 antibodies used in cancer therapy block PD-1 interaction with its ligand molecule, PD-1 agonists capable of inducing the function of PD-1 by binding to PD-1 are highly promising as drugs for positive immunosuppression.

The present invention aims at searching for novel physiological activity of agonist antibody to PD-1, and finding a novel use for the agonist antibody based on such physiological activity.

### MEANS TO SOLVE THE PROBLEM

There are a number of inflammatory diseases caused by excessive immune response, and a novel and more effective treatment is demanded in many cases. Targeting an immune regulatory molecule PD-1, the present inventors have developed a treatment effective for inflammatory diseases such as type I allergy and eosinophilic diseases mediated by Th2 cells. As a result of screening of anti-human PD-1 antibodies, the present inventors have found a large number of anti-human PD-1 antibodies which have the agonist activity of inducing the immunosuppressive activity of PD-1. Of these anti-PD-1 agonist antibodies, especially those with a strong agonist activity were found to be effective in suppressing differentiation to Th2 cells. Based on this finding, the present inventors have administered an anti-PD-1 agonist antibody to a model mouse having allergic asthma included in a Th2 type disease. As a result, a remarkable anti-inflammatory effect was observed together with suppression of Th2 type immune response. These results show that PD-1 agonists are useful as an anti-allergic drug or a therapeutic for eosinophilic diseases.

The present invention has been achieved based on these findings, and a summary thereof is described as below.
(1) A pharmaceutical composition for treating or preventing Th2-mediated diseases, the composition comprising an effective amount of a PD-1 agonist.
(2) The pharmaceutical composition of (1) wherein the PD-1 agonist is an anti-PD-1 agonist antibody or a functional fragment thereof.
(3) The pharmaceutical composition of (1) or (2), wherein the Th2-mediated disease is type I allergy.
(4) The pharmaceutical composition of (1) or (2), wherein the Th2-mediated disease is an eosinophilic disorder.
(5) The pharmaceutical composition of (1) or (2), wherein the Th2-mediated disease is a disease selected from the group consisting of bronchial asthma, atopic dermatitis, allergic rhinitis, drug allergy, food allergy, anaphylaxis, allergic conjunctivitis, urticaria, eosinophilic sinusitis, eosinophilic gastrointestinal diseases and allergic bronchopulmonary aspergillosis.
(6) A method of preventing and/or treating Th2-mediated diseases, comprising administering an effective amount of a PD-1 agonist to a subject.
(7) APD-1 agonist for use in preventing and/or treating Th2-mediated diseases.
(8) Use of a PD-1 agonist for preventing and/or treating Th2-mediated diseases.
(9) APD-1 agonist and use thereof for suppressing IgE production by suppression of Th2 type cytokines.
(10) APD-1 agonist and use thereof for suppressing activation of eosinophils by suppression of Th2 type cytokines.

### EFFECT OF THE INVENTION

The present invention makes it possible to suppress the functional differentiation of naive CD4⁺ T cells to Th2 cells. Consequently, it becomes possible to treat or prevent Th2-mediated diseases.

The present specification encompasses the contents disclosed in the specification and/or the drawings disclosed in Japanese Patent Application No. 2020-90526 based on which the present patent application claims priority.

### BRIEF DESCRIPTION OF DRAWINGS

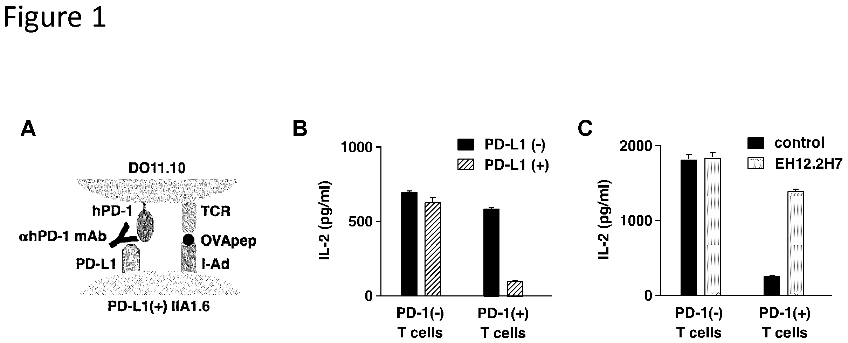

[Fig. 1] Evaluation system for suppressive activity against cytokine production from T cells by PD-1 stimulation. (A) DO11.10 T cell hybridomas rendered to express human PD-1 (hPD-1) and IIA1.6 B cell lymphoma cells were used. DO11.10 T cell hybridomas are activated in response to OVA₃₂₃₋₃₃₉ peptide presented by MHC class II molecule (I-A^{d}) of IIA1.6 cells, but the activation is suppressed when stimulation to PD-1 occurs. (B) When interaction with PD-L1 on IIA1.6 cells occurs, IL-2 production from the human PD-1 expressing DO11.10 T cell hybridomas is suppressed. In the case where PD-1 or PD-L1 is not expressed, suppression of IL-2 is not observed. (C) When EH12.2H7, a blocking antibody to human PD-1, is added to the evaluation system, suppressive effect by PD-L1 is cancelled. With this experimental system, detection of anti-PD-1 antibodies having an immunoregulatory activity is possible.

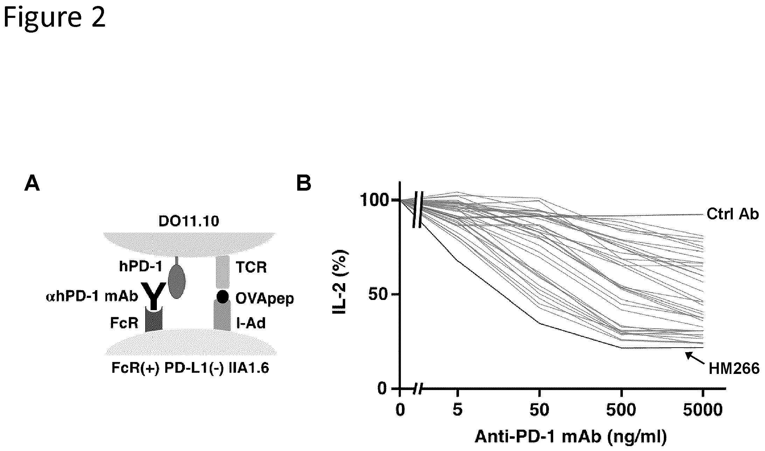

[Fig. 2] Evaluation of agonistic activity of anti-human PD-1 antibodies. (A) An evaluation system combining DO11.10 T cell hybridomas rendered to express human PD-1, and IIA1.6 B cell lymphoma cells rendered to express not PD-L1 but FcyRIIB was used. With this system, screening of anti-human PD-1 antibodies was performed using suppressive activity against IL-2 production as an indicator. (B) As a result of evaluation, about 30 clones having an immunosuppressive activity were obtained, including those with high activity and those with low activity. Among these anti-human PD-1 antibodies, only the clones having an immunosuppressive activity are shown in the graph.

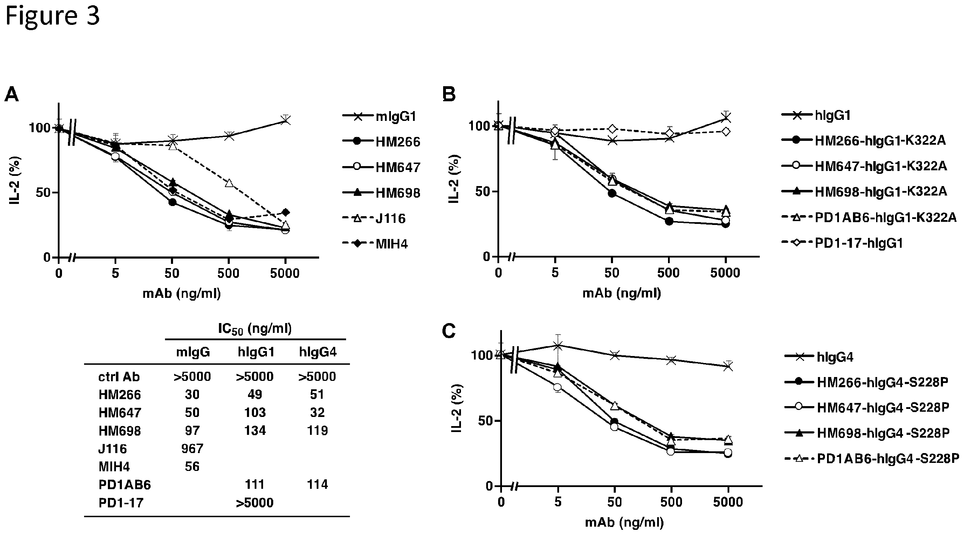

[Fig. 3] Comparison between the activities of anti-human PD-1 agonist antibodies. (A) Among the anti-human PD-1 antibodies found to have an immunosuppressive activity, those having relatively high activity were selected and IC₅₀ was determined. Not only the novel antibodies, but also commercially available antibodies (J116, MIH4) which were found to have agonistic activity are all mouse IgG. Based on the suppression curve of IL-2 production in an antibody concentration-dependent manner by these antibodies, the concentration at which 50% suppression was reached was determined, taking the state of no IL-2 production as 0%. (B, C) Chimeric antibodies with the Fv region of the novel antibodies and the Fc region of human IgG1-K322A (B) or human IgG4-S228P (C) were prepared. In addition to these chimeric antibodies, known PD-1 antibodies reported as agonist antibodies [PD1AB6 and PD1-17; Japanese Unexamined Patent Application Publication (Translation of PCT Application) Nos. 2018-533973 and 2006-521783] were also examined. IC₅₀ values of these antibodies were examined based on the antibody concentration-dependent suppression curve of IL-2 production.

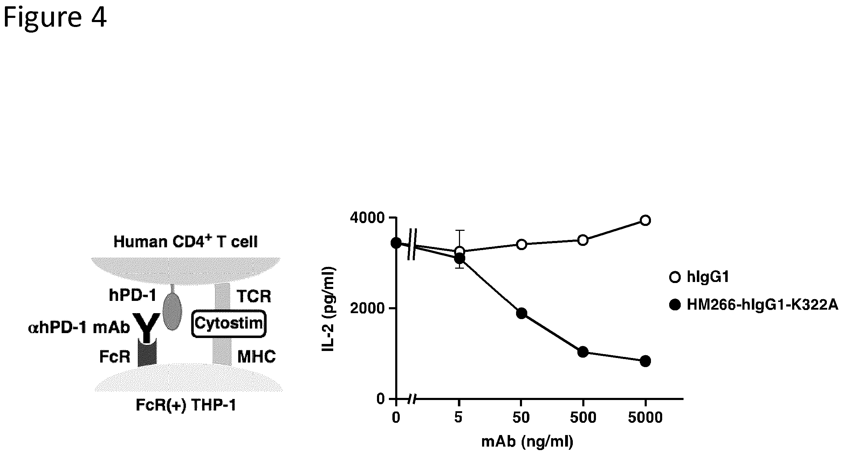

[Fig. 4] Immunosuppressive activity of anti-human PD-1 agonist antibodies on human T cells. Primary cultured human CD4⁺ T cells were stimulated with anti-CD3 antibody for 3 days for activation. Then, THP-1 cells rendered to express human FcyRIIB were mixed therewith, and the T cells were stimulated with CytoStim (Miltenyi Biotec). Anti-human PD-1 agonist antibody was added to this culture system, and suppression of cytokine production was observed.

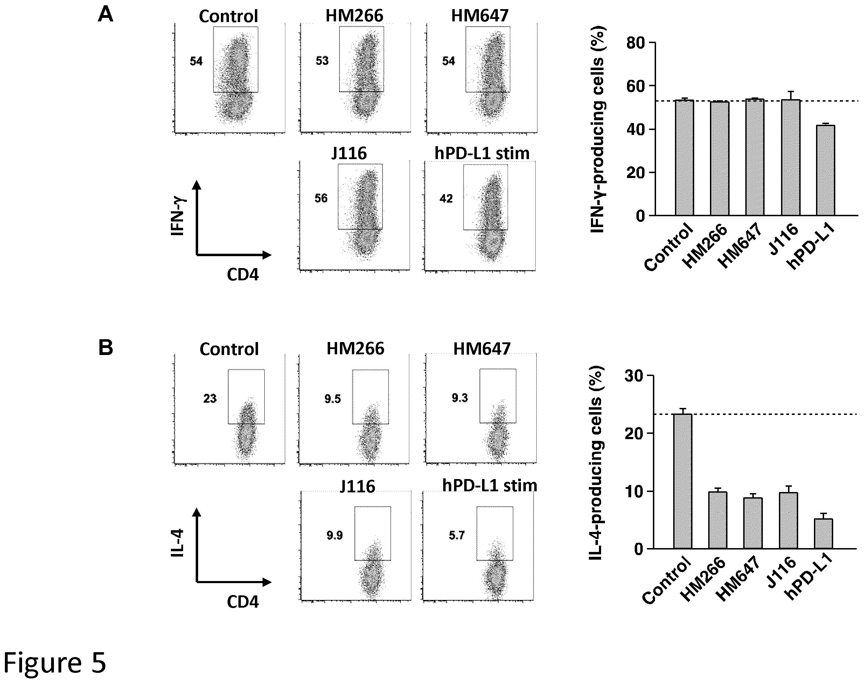

[Fig. 5] Suppression of Th2 cell induction by PD-1 stimulation. CD4⁺ CD62L⁺ cells prepared from the spleen of DO11.10 mice were stimulated with OVA₃₂₃₋₃₃₉ using PD-L 1 (-) FcγRIIB(+) IIA1.6 cells as antigen-presenting cells. At that time, cytokines and anti-cytokine antibodies were added to selectively induce Th1 or Th2 cells. IFN-γ and IL-4 productions after re-stimulation were analyzed by intracellular staining. Then, induction ratios of Th1 and Th2 cells were examined. When Th1 and Th2 cells were induced in the presence of anti-PD-1 agonist antibody, the ratio of Th1 cells which is represented by IFN-γ production did not show a significant change (A), but the ratio of Th2 cells which produce IL-4 was largely suppressed (B). Functional differentiation to Th2 was especially sensitive to PD-1 stimulation, and it is possible that Th1/Th2 balance can be artificially modified by the use of anti-PD-1 agonist antibody. When PD-1 was stimulated with PD-L1(+) IIA1.6 cells, similar changes as seen with anti-PD-1 agonist antibody were also observed.

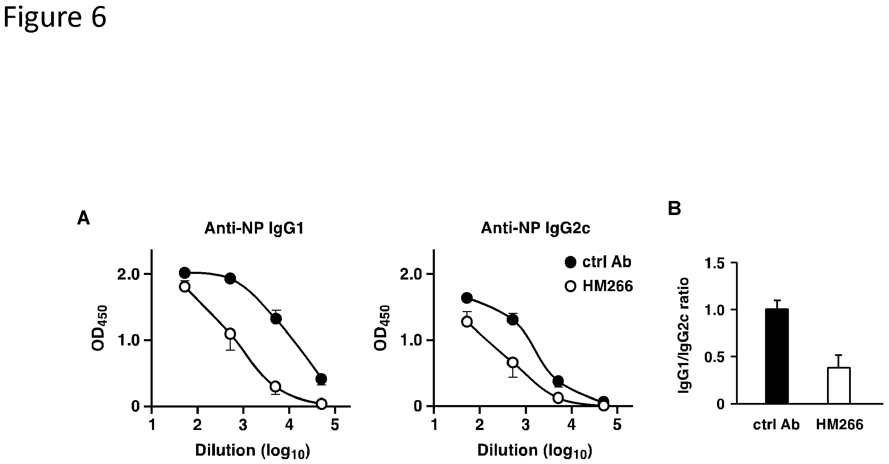

[Fig. 6] Antibody production in antigen-immunized mice and effect of anti-PD-1 agonist antibody against the antibody production. Briefly, human PD-1 knock-in mice were immunized with NP-OVA (4-hydroxy-3-nitrophenylacetyl hapten-conjugated ovalbumin). Simultaneously, administration of anti-PD-1 agonist antibody (HM266) was started. HM266 was also administered 3 days and 7 days after the start of immunization. Ten days after the immunization, hapten-specific antibody titers in the blood were measured, and compared between IgG subclasses. (A) Blood antigen-specific antibody titer 10 days after the immunization. Although the administration of HM266 decreased concentrations of both IgG1 type and IgG2c type antibodies, suppressive action on IgG1 type antibody was especially remarkable. (B) IgG1 / IgG2c ratio of the blood antigen-specific antibody titer 10 days after the immunization. HM266 administration decreased IgG1 / IgG2c ratio.

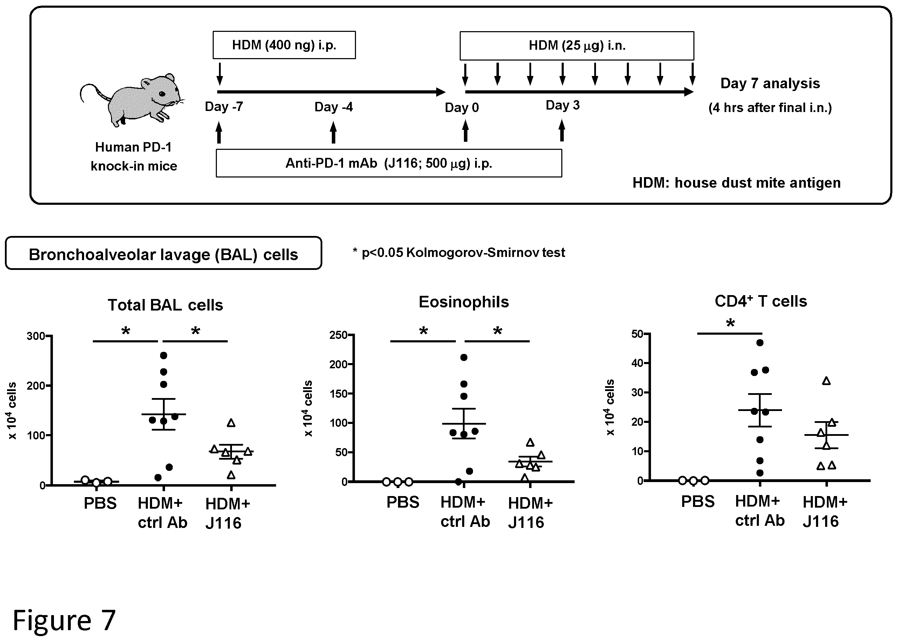

[Fig. 7] Induction of allergic asthma in mice and anti-inflammatory action of J116. Allergic asthma was induced by sensitizing human PD-1 knock-in mice with house dust mite antigen (HDM) and nasally administrating the same antigen from one week later. Seven days after the start of daily HDM nasal administration, infiltration of inflammatory cells into the alveoli was examined in the bronchoalveolar lavage fluid. Induction of allergic asthma increased the total number of infiltrated inflammatory cells, most of which were eosinophils and CD4⁺ T cells. However, the administration of J116 in this inflammation model remarkably suppressed the alveolar infiltration of inflammatory cells, especially eosinophils.

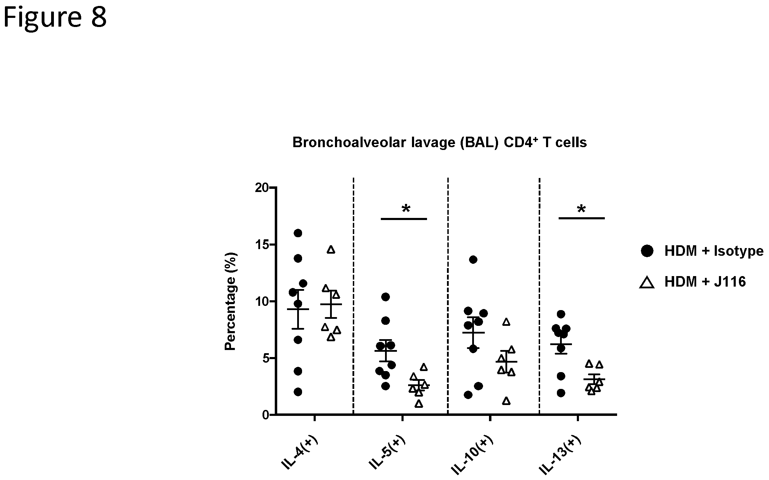

[Fig. 8] Suppression of Th2 type cell increase in allergic asthma mouse by J116. Allergic asthma was induced as described in Fig. 7, and 7 days after the start of daily HDM nasal administration, bronchoalveolar lavage fluid was collected. Cytokine production in CD4⁺ cells infiltrating into the alveoli was examined by intracellular staining. As a result, a significant decrease in IL-5 and IL-13 producing cells was observed in J116-treated mice.

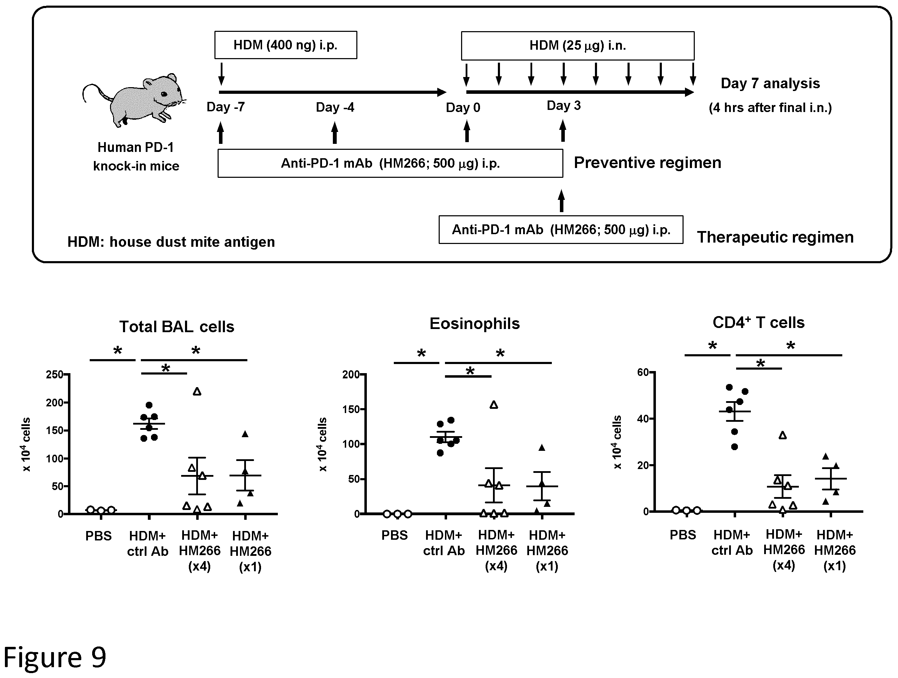

[Fig. 9] Induction of allergic asthma in mice and anti-inflammatory action of HM266. Allergic asthma was induced in human PD-1 knock-in mice by exposure to house dust mite antigen as described in Fig. 7. At this time, HM266 was administered to mice, and suppression of inflammation was observed. Four times administration of HM266 (HM266 (x4)) in the same schedule as in Fig. 7 suppressed the infiltration of eosinophils and CD4⁺ T cells into the alveoli. Furthermore, when HM266 was administered only once (HM266 (x1)) 3 days after the induction of inflammation in the lung tissue in order to observe the therapeutic effect, the alveolar infiltration of inflammatory cells was also suppressed.

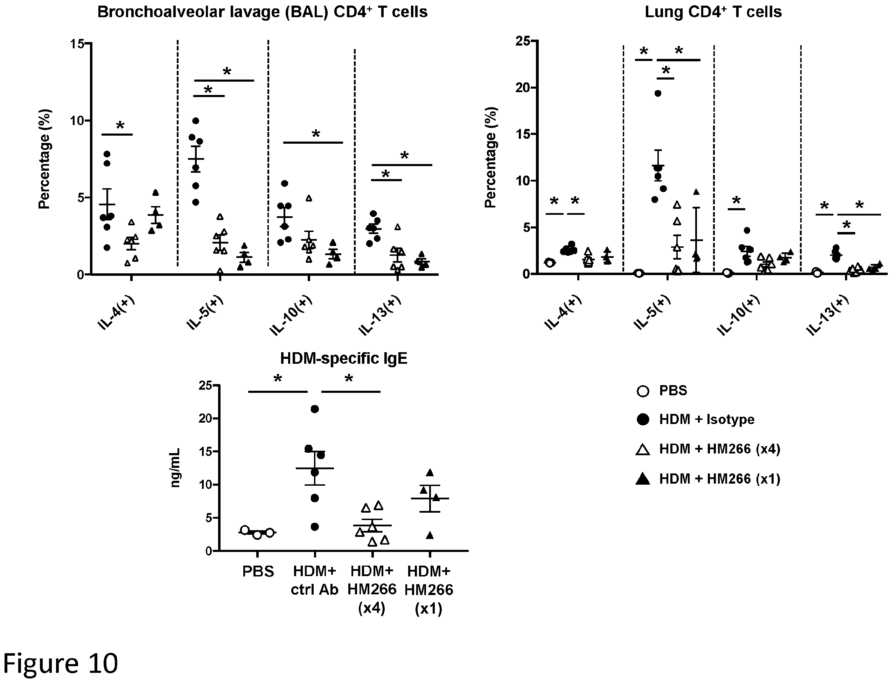

[Fig. 10] Anti-inflammatory effects of HM266 on allergic asthma and suppression of Th2-type immune response. Allergic asthma was induced as described in Fig. 9, and the bronchoalveolar lavage fluid and lung tissue were collected. Cytokine production in CD4⁺ cells infiltrating into the alveoli or lung tissue was examined by intracellular staining. Decreases in IL-4, IL-5, IL-10 and IL-13-producing cells were observed in the group receiving HM266 four times (HM266 (x4)). Furthermore, blood concentration of IgE specific to house dust mite antigen was also significantly decreased by the administration of HM266. A similar trend was also observed when HM266 was administered only once (HM266 (x1)) 3 days after the start of inflammation induction in the lung, suggesting that anti-human PD-1 agonist antibody is useful for treatment of allergic diseases.

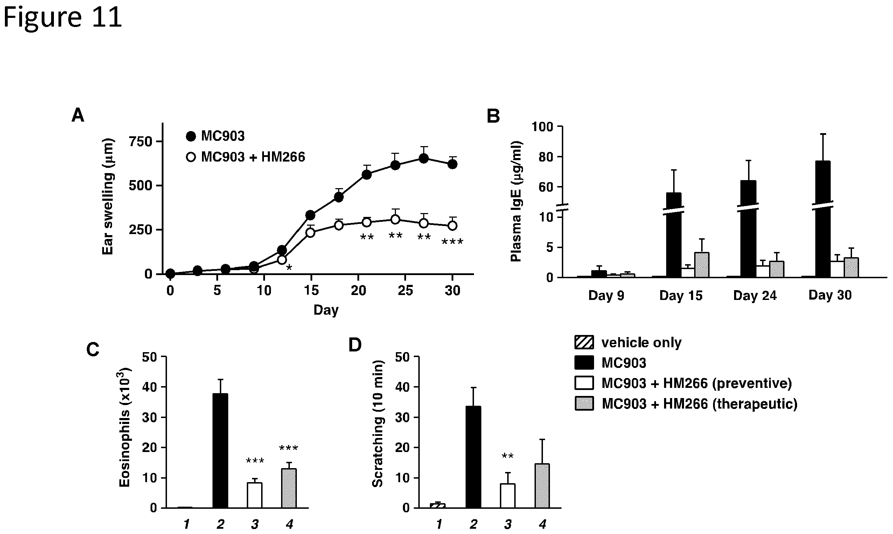

[Fig. 11] Anti-inflammatory effect of HM266 on atopic dermatitis. Allergic dermatitis was induced in human PD-1 knock-in mice by applying MC903 to the auricle every three days. (A) Swelling in the auricle was significantly suppressed by simultaneous treatment with anti-PD-1 agonist antibody (HM266) and MC903. (B) Time-dependent changes of blood IgE concentration by MC903 administration. Repeated MC903 application remarkably increased IgE concentration; however, simultaneous administration of HM266 (preventive administration) greatly decreased IgE levels. Furthermore, even in a therapeutic regimen where administration of HM266 starts 12 days after occurrence of swelling in the auricle, IgE concentration also decreased remarkably. (C, D) As a result of HM266 administration, an apparent suppressive effect was observed in the number of eosinophils infiltrating into the auricle tissue (C) and the scratching behavior of mice (D). Groups 1, 2, 3 and 4 appearing in (C) and (D) in Fig. 11 represent vehicle only, MC903, MC903+HM266 (preventive) and MC903+HM266 (therapeutic), respectively.

### BEST MODES FOR CARRYING OUT THE INVENTION

Hereinbelow, embodiments of the present invention will be described in detail.

The present invention provides a pharmaceutical composition for treating or preventing Th2-mediated diseases, the composition comprising an effective amount of a PD-1 agonist, especially anti-PD-1 agonist antibody. The present invention also provides a method of preventing and/or treating Th2-mediated diseases, comprising administering an effective amount of a PD-1 agonist to a subject. The present invention further provides a PD-1 agonist for use in preventing and/or treating Th2-mediated diseases. Still further, the present invention provides use of a PD-1 agonist for preventing and/or treating Th2-mediated diseases.

PD-1 (Programmed cell death 1) is a receptor expressed on the surface of activated T cells. On the other hand, PD-L1 and PD-L2 which are ligands of PD-1 are expressed on the surface of antigen-presenting cells. Once PD-L1 or PD-L2 is bound to PD-1, the immunoreactivity of T cells is suppressed via signal transduction pathways.

The term "PD-1 agonist" means a substance which, like PD-L1, shows an agonistic action on PD-1 molecule (i.e., enhancement of activation signals). The pharmaceutical composition of the present invention comprises a PD-1 agonist as an active ingredient. PD-1 agonist is not particularly limited. Any PD-1 agonist may be used, for example, antibodies or antigen-binding fragments thereof, solubilized PD-L1/L2 (such as Fc fusion proteins), peptides, nucleic acid molecules, other compounds, PD-1 ligand-expressing cells whose expression of PD-L1/L2 is artificially enhanced, and so on. Preferably, PD-1 agonist is an antibody (anti-PD-1 agonist antibody) or a functional fragment thereof.

The present inventors evaluated the agonistic activity of anti-human PD-1 antibodies under stimulation with OVA₃₂₃₋₃₃₉ peptide (ovalbumin-derived peptide antigen) using a system combining DO11.10 T cell hybridomas rendered to express human PD-1 and IIA1.6 B cell lymphoma cells rendered to express not PD-L1 but FcyRIIB. As a result, a plurality of clones having an immunosuppressive activity were obtained, including those with high activity (inhibitory activity on IL-2 production) and those with low activity (see Example described later). In this assay system, J116, a commercially available anti-human PD-1 monoclonal antibody, exhibited an inhibitory activity on IL-2 production, and IC₅₀ was approximately 1000 ng/ml. Therefore, to take the above assay system as an example, an antibody exhibiting an inhibitory activity on IL-2 production comparable to or higher than that of J116 (i.e. an IC₅₀ value comparable to or lower than the IC₅₀ value of J116) under conditions where the IC₅₀ of J116 is calculated to range from 50 ng/ml to 5000 ng/ml in terms of inhibitory activity on IL-2 production can be described as an "anti-PD-1 agonist antibody".

With respect to PD-1 agonists other than antibodies, those substances may be used which are scientifically recognized to have an inhibitory activity on IL-2 production comparable to or higher than that of 1116.

The anti-PD-1 agonist antibody or a functional fragment thereof may bind to only domain #7 of human PD-1 (domain spanning from amino acid No. 38 to No. 48 in the amino acid sequence as shown in SEQ ID NO: 24) or may also bind to other domains of human PD-1 such as domain #6 (domain spanning from amino acid No. 109 to No. 120 in the amino acid sequence as shown in SEQ ID NO: 24) or domain #1 (domain spanning from amino acid No. 129 to No. 139 in the amino acid sequence as shown in SEQ ID NO: 24). Preferably, the antibody or a functional fragment thereof binds to only domain #7 of human PD-1. When the binding capacity of an anti-human PD-1 antibody is decreased by replacing domain #7 of human PD-1 as shown in SEQ ID NO: 24 with the amino acid sequence of the corresponding domain in the amino acid sequence of mouse PD-1 (SEQ ID NO: 25), the anti-human PD-1 antibody can be defined as binding to domain #7 of human PD-1 as shown in SEQ ID NO: 24. Here, the binding to domain #7 is judged independently from the binding to other domains of human PD-1. The binding to domains of human PD-1 other than #7 is defined in the same manner. The amino acid sequence of human PD-1 is registered at the NCBI database under accession number: NP_005009.2, and the amino acid sequence of mouse PD-1 at the NCBI database under accession number: NP_032824.1. Preferably, the anti-PD-1 agonist antibody or a functional fragment thereof also binds to substituted mouse PD-1 (Mouse PD-1 (hu38-48)) in which the amino acid sequence of domain #7 of mouse PD-1 is replaced with the amino acid sequence of domain #7 of human PD-1 and/or mutant human PD-1 (R143A point mutant) in which arginine at position 143 of human PD-1 is mutated to alanine.

Commercially available anti-human PD-1 monoclonal antibodies MIH4 and J116 exhibited an activity as PD-1 agonist in Example described later. Further, anti-human PD-1 monoclonal antibodies MH266, HM647 and HM698 which were newly prepared by the present inventors exhibited a favorable agonistic activity. These antibodies are useful as anti-PD-1 agonist antibody and may be used as an active ingredient of the pharmaceutical composition of the present invention. MIH4, J116, HM266 and HM268 bind to domain #7 of human PD-1, and HM647 to domains #6 and #7 of human PD-1.

The amino acid and nucleotide sequences of the heavy chain variable regions and the light chain variable regions of HM266, HM647 and HM698 are shown in the table below as accompanied by respective sequence ID numbers.

| | | Heavy Chain | | Light Chain | |
|---|---|---|---|---|---|
| | | Variable Region | Constant Region | Variable Region | Constant Region |
| HM266 | Amino Acid Sequence | SEQ ID NO: 1 | SEQ ID NO: 13 (IgG1 CH) | SEQ ID NO: 3 | SEQ ID NO: 15 (Ig κ CL) |
| | Nucleotide Sequence | SEQ ID NO: 2 | SEQ ID NO: 14 (IgG1 CH) | SEQ ID NO: 4 | SEQ ID NO: 16 (Ig κ CL) |
| HM647 | Amino Acid Sequence | SEQ ID NO: 5 | SEQ ID NO: 13 (IgG1 CH) | SEQ ID NO: 7 | SEQ ID NO: 15 (Ig κ CL) |
| | Nucleotide Sequence | SEQ ID NO: 6 | SEQ ID NO: 14 (IgG1 CH) | SEQ ID NO: 8 | SEQ ID NO: 16 (Ig κ CL) |
| HM698 | Amino Acid Sequence | SEQ ID NO: 9 | SEQ ID NO: 13 (IgG1 CH) | SEQ ID NO: 11 | SEQ ID NO: 15 (Ig κ CL) |
| | Nucleotide Sequence | SEQ ID NO: 10 | SEQ ID NO: 14 (IgG1 CH) | SEQ ID NO: 12 | SEQ ID NO: 16 (Ig κ CL) |

HM266, HM647 and HM698 may be prepared using hybridomas. Alternatively, they may be prepared as recombinant antibodies by genetic engineering techniques. Briefly, a DNA encoding a heavy chain gene and a light chain gene of an antibody of interest is synthesized, inserted into an expression vector (e.g., plasmid), and then introduced into host cells (e.g., CHO cells, HEK cells, etc.). By culturing the host cells, a recombinant antibody can be obtained from the culture. Codon optimization is preferable in synthesizing a DNA encoding a heavy chain gene and a light chain gene of the antibody.

The PD-1 agonist antibody may be either a polyclonal antibody or a monoclonal antibody. Preferably, the agonist antibody is a monoclonal antibody. Polyclonal antibodies may be prepared by injecting an antigen (PD-1) into an animal to thereby produce antibodies in the blood, repeating this process, collecting the blood (plasma, serum) and then purifying antibodies therefrom. Monoclonal antibodies may be prepared by injecting an antigen (PD-1) into an animal, collecting antibody-producing B cells from the spleen or lymph nodes, artificially fusing the B cells to immortalized cancer cell (myeloma) to prepare hybridomas, selecting monoclonal antibody-producing cells from the hybridomas, and allowing the cells to produce monoclonal antibodies. As regards animals for immunization, various mammals, birds and fishes such as rat, hamster, guinea pig, chicken, goat, sheep, donkey, llama, shark, or the like may be enumerated in addition to mouse and rabbit. It is also possible to prepare monoclonal antibodies as recombinant antibodies by genetic engineering techniques. A monoclonal antibody may be any one of the following: antibody of non-human animals (various mammals, birds and fishes such as mouse, rabbit, rat, hamster, guinea pig, goat, sheep, donkey, llama, camel, chicken, ostrich, shark, etc.), chimeric antibody, humanized antibody, and full human antibody. For example, the variable region (Fv) of anti-PD-1 agonist antibody may be the Fv region of an antibody derived from an animal other than human (for example, mouse, rabbit, rat, hamster, guinea pig, goat, sheep, donkey, llama, camel, chicken, ostrich, shark, etc.) or it may be a humanized Fab region of the heavy chain and/or the light chain of an antibody derived from a non-human animal. Humanization may be performed by transplanting the CDRs of VH and VL of non-human animal-derived antibodies into the frameworks of VH and VL of human antibodies (Nature, 332, 323-327, 1988). Humanized antibody may be one in which CDR sequences are retained. Alternatively, humanized antibody may also be one in which antigen binding is improved by, for example, identifying the amino acid residues directly involved in binding to antigen, the amino acid residues interacting with CDRs and the amino acid residues involved in retaining the three-dimensional structure of CDRs, and replacing these residues with amino acid residues of non-humanized antibodies (MABS, 8(7), 1302-1318, 2016). In Example described later, chimeric antibodies were prepared in which the variable region of novel antibodies (HM266, HM647 and HM698) was ligated to the constant region of human IgG1 or human IgG4. The amino acid sequence and the nucleotide sequence of the heavy chain constant region of human IgG1 (IgG1-K322A) used in the preparation of the chimeric antibodies are shown in SEQ ID NOS: 17 and 18, respectively. The amino acid sequence and the nucleotide sequence of the heavy chain constant region of human IgG4 (IgG4-S228P) used therein are shown in SEQ ID NOS: 19 and 20, respectively. The amino acid sequence and the nucleotide sequence of the light chain (κ chain: Igκ) constant region of human immunoglobulin used therein are shown in SEQ ID NOS: 21 and 22, respectively.

The anti-PD-1 agonist antibody may undergo functional modifications. Techniques for functional modification of antibodies include, but are not limited to, introduction of amino acid mutations, subclass substitution, antibody-drug complexes, sugar chain-modified antibodies, and combinations thereof. In introduction of amino acid mutations, it is preferred that mutations be introduced which would improve the affinity to the Fc receptor of the human or non-human animal as a subject to be administered with the anti-PD-1 agonist antibody. The Fc receptor is preferably Fcγ receptor, more preferably FcyRII, and still more preferably FcyRIIB. The binding capacity (binding affinity) of the anti-PD-1 agonist antibody to human Fc receptors may be evaluated by an human Fc receptor binding affinity test using surface plasmon resonance technology with Biacore 8K (Cytiva) or a flow cytometer-based binding test for human Fc receptor-expressing cell line. When measured by the flow cytometer-based binding test for human Fc receptor-expressing cell line, the affinity of the anti-PD-1 agonist antibody to human FcyRIIB can be expressed as a GMFI ratio to an antibody having the Fc region of human IgG1-K322A (reference antibody). Under measuring conditions where a GMFI value without antibody addition is about 40 and a GMFI value with addition of the reference antibody is 300-1500, the anti-PD-1 agonist antibody shows 2-fold or more GMFI, preferably 5-fold or more GMFI, and more preferably 20-fold or more GMFI as compared with the case where the reference antibody having the same Fv region is added. When measured by the Fc receptor binding affinity test using surface plasmon resonance technology, the affinity of the anti-PD-1 agonist antibody to human FcyRIIB can be expressed as an equilibrium dissociation constant (K_{D}) ratio to an antibody having the Fc region of human IgG1-K322A (reference antibody). The anti-PD-1 agonist antibody has 1.5-fold or more affinity than the reference antibody, preferably 2-fold or more affinity than the reference antibody, and more preferably 2.5-fold or more affinity than the reference antibody.

The anti-PD-1 agonist antibody may be one having the Fc region of a human antibody (for example, IgG1, IgG4, etc.) (a region spanning from amino acid No. 100 to No. 330 of the amino acid sequence of IgG1 as shown in SEQ ID NO: 17; a region spanning from amino acid No. 100 to No. 327 of the amino acid sequence of IgG4 as shown in SEQ ID NO: 19; etc.) and, preferably, the Fc region of the human antibody is modified so that the affinity to human Fc receptors is improved. By modifying the Fc region of an antibody, its affinity to human Fc receptors can be improved. Alternatively, the affinity of an antibody to human Fc receptors can also be improved by defucosylation.

As a means to improve the affinity of the anti-PD-1 agonist antibody to human Fc receptors, a mutation(s) may be introduced, for example, to at least one position, or preferably a combination of positions, selected from the group of amino acid positions consisting of 236, 268, 239, 328, 332, 233, 237, 238, 271, 330, 267, 326, 234, 323 and 296 (according to Kabat's EU numbering; hereinafter, the same shall apply) in the amino acid sequence of the Fc region of human IgG1. Further, such mutation(s) may be combined with defucosylation.

When mutation(s) is/are to be introduced into the Fc region of human IgG1, they may be combined with other mutations, as exemplified by K322A (a mutation known to suppress complement-dependent cytotoxicity (CDC) activity by decreasing the binding of complement C1q) or E293A (a mutation known to suppress ADCC activity by decreasing the binding to FcγRIIIA). In Example described later, a heavy chain constant region of human IgG1 having Lys322Ala (an amino acid mutation known to suppress CDC activity) was used. However, it is understood that these mutations do not have a significant effect in the testing system disclosed in Example described later using antibodies having a human heavy chain constant region.

As an exemplary means to improve the binding capacity to human Fc receptors, a mutation may be introduced at any one or more of the following positions: 236, 239, 268, 328, or 332 in the amino acid sequence of the Fc region of human IgG4, for example. Preferably, a mutation may be introduced at a combination of those positions. Further, such mutations may be combined with defucosylation.

When a mutation is to be introduced into the Fc region of human IgG4, it may be combined with other mutations such as S228P that is known to be effective for improving antibody stability. In Example described later, a heavy chain constant region of human IgG4 having Ser228Pro (an amino acid mutation known to improve antibody stability) was used. However, it is understood that these mutations do not have a significant effect in the testing system disclosed in Example described later using antibodies having a human heavy chain constant region.

The anti-PD-1 agonist antibody may also be one that comprises a Fab region in which each of the Fab regions of a heavy chain and a light chain of an antibody (showing PD-1 agonist activity) derived from a non-human animal (e.g., mouse) has been humanized, and the Fc region of an Fc region-modified form of human IgG1 or IgG4.

As used herein, a "functional fragment" of the anti-PD-1 agonist antibody refers to a protein fragment derived from the antibody, which is capable of binding to PD-1; a fusion protein comprising the protein fragment; or the like. Specific examples of functional fragment include, but are not limited to, bi-specific antibodies, and low molecular weight antibodies (such as scFv, Fv, F(ab')2, Fab', Fab, diabody, etc.). For example, a functional fragment may also be a molecule which has one or more of the scFv, Fv, F(ab')2, Fab', Fab, etc. of the anti-PD-1 agonist antibody and which yet is capable of binding to Fc receptors. Other exemplary functional fragments include, but are not limited to, an antibody-drug complex composed of the anti-PD-1 agonist antibody and a drug; a polypeptide having the scFv of the anti-PD-1 agonist antibody and the scFv of an anti-Fc receptor antibody; and a fusion protein having the scFv of the anti-PD-1 agonist antibody and an Fc region. When a functional fragment has an Fc region (for example, when a functional fragment is a fusion protein of the scFv of the anti-PD-1 agonist antibody and an Fc region), it is desirable that the Fc region is the Fc region of the Fc region-modified forms described in the present specification. The protein improvement and codon optimization techniques mentioned above may also be applied to functional fragments.

The anti-PD-1 agonist antibody may be an immunoglobulin molecule, preferably an immunoglobulin molecule of the animal species in which the antibody is to be used. The immunoglobulin molecule may be a molecule of any class. In human, IgG is preferable. IgG of any subclass may be used but IgG1 or IgG4 is preferable.

The affinity of the anti-PD-1 agonist antibody to PD-1 may be 10⁻⁷ M or less, preferably 10⁻⁸ M or less, in terms of equilibrium dissociation constant (K_{D}). Equilibrium dissociation constant is measured preferably by the surface plasmon resonance (SPR) method. Simply, the affinity of the anti-PD-1 agonist antibody to PD-1 can also be measured by flow cytometry using the concentration dependency of binding to PD-1 expressing cells.

The present inventors have found that the anti-PD-1 agonist antibody is capable of suppressing Th2 cell induction by PD-1 stimulation (see Example described later; Fig. 5). Th2 cells produce IL-4, IL-5 and IL-13, and it is through these cytokines that immune functions are regulated.

The pharmaceutical composition of the present invention may be used for treating or preventing Th2-mediated diseases. As used herein, the term "Th2-mediated diseases" means those diseases which are associated with immune responses induced by Th2 type cytokine (such as IL-4, IL-5 or IL-13)-producing cells (e.g., Th2 cells) acting as a key player. Specifically, inflammatory diseases caused by type I allergic response, or eosinophilic diseases [diseases in which functions regulated by Th2 type cytokines (e.g., proliferation of eosinophils, release of granular protein, migration, etc.) are involved in the pathology] are Th2-mediated diseases. Briefly, diseases which are considered to be directly associated with Th2 type immunity may be enumerated as Th2-mediated diseases. Specific examples of such diseases include, but are not limited to, bronchial asthma, atopic dermatitis, allergic rhinitis (such as pollinosis), drug allergy, food allergy, anaphylaxis, allergic conjunctivitis, urticaria, eosinophilic sinusitis, eosinophilic gastrointestinal diseases and allergic bronchopulmonary aspergillosis.

The pharmaceutical composition of the present invention may be administered to subjects (human or non-human animal) systemically or topically by an oral or parenteral route.

The pharmaceutical composition of the present invention may comprise an effective amount of the PD-1 agonist, and may be formulated into a preparation by mixing, dissolving, emulsifying, encapsulating, lyophilizing, etc. with a pharmaceutically acceptable carrier.

The pharmaceutical composition of the present invention is suitable for either oral or parenteral administration. Preferable preparations for oral administration include, but are not limited to, liquids which have an effective amount of the anti-PD-1 agonist antibody dissolved in a diluent such as water or physiological saline; capsules, granules, powders or tablets containing an effective amount of said antibody as a solid or granules; suspensions which have an effective amount of said antibody suspended in an appropriate dispersion medium; and emulsions prepared by first dissolving an effective amount of said antibody in a solution which is then dispersed and emulsified in an appropriate dispersion medium.

For parenteral administration, the anti-PD-1 agonist antibody may be formulated into injections, suspensions, emulsions, creams, ointments, inhalants, suppositories or the like, together with pharmaceutically acceptable solvents, excipients, binders, stabilizers, dispersants and the like. In the formulation of injections, the antibody of the present invention may be dissolved in an aqueous solution (preferably Hanks solution or Ringer solution) or a physiologically compatible buffer such as physiological saline buffer. Further, the pharmaceutical composition of the present invention may take the form of suspension, solution or emulsion, etc. in an oily or aqueous vehicle. Alternatively, the antibody of the present invention may be produced in the form of a powder, which may be prepared into an aqueous solution or suspension with sterile water or the like before use. For administration by inhalation, the antibody of the present invention may be pulverized to prepare a powder mixture with an appropriate base such as lactose or starch. Suppository formulations may be prepared by mixing the antibody of the present invention with a routinely used suppository base such as cocoa butter. Further, the therapeutic of the present invention may be formulated as a sustained release preparation by encapsulating in a polymer matrix or the like.

The dose may be about 0.1 to 100 mg/kg (body weight) per human adult, and this dose may be administered once or multiple times at intervals of about 1 day to 6 months. Administration route may be either oral or parenteral. Parenteral administration includes, but are not limited to, intravenous, intramuscular, subcutaneous, rectal, nasal, intraoral, and transdermal administration.

Non-human animals as the subject to be administered with the pharmaceutical composition of the present invention include mammals (such as dog, cat, bovine, swine, horse, etc.) and birds.

The pharmaceutical composition of the present invention may be used alone. Alternatively, the composition may be used in combination with other therapeutics such as antihistamines, antiallergics, vasoconstrictive nasal sprays, steroids, small molecule immunosuppressants (cyclosporine, tacrolimus, etc.), antibody preparations (e.g., anti-IgE antibody, anti-IL-4 antibody, anti-IL-5 antibody, anti-IL-13 antibody, anti-IL-4 receptor antibody, anti-IL-5 receptor antibody, anti-IL-22 antibody, anti-IL-25 antibody, anti-IL-33 antibody, anti-TSLP antibody, anti-BAFF antibody, etc.), and recombinant soluble fusion proteins (CTLA-4-Ig, etc.). From such a combined use, synergism of drug efficacies can be expected.

### EXAMPLES

Hereinbelow, the present invention will be described more specifically with reference to the following Example.

### [Example 1]

### Methods

### Commercially Available Antibodies

Anti-human PD-1 antibody (clone: EH12.2H7, BioLegend), anti-human PD-1 antibody (clone: J116, Invitrogen), anti-human PD-1 antibody (clone: MIH4, Invitrogen), control mouse IgG1 (clone: MOPC-21, BioLegend), control human IgG1 (clone: QA16A12, BioLegend), and control human IgG4 (clone: QA16A15, BioLegend)

### Acquisition and Purification of Novel Antibodies

Techniques for producing anti-human PD-1 mouse monoclonal antibody are known in the art. For example, the method described in Goding, Monoclonal Antibodies: Principles and Practice, pp. 59-103 (Academic Press, 1986) was used. As immunization hosts, A/J and BALB/c mice were used. As immunogens, a plasmid vector (15-50 µg) expressing the full length or a mutant of human PD-1 protein (NCBI accession number: NP_005009.2), a fusion protein of recombinant human PD-1 extracellular domain and human IgG1-Fc region (25 µg), and 293T cells so engineered to transiently express human PD-1 or a point mutant thereof (5 x 10⁷ cells) were used. One of these immunogens was intramuscularly, intradermally, intraperitoneally or intravenously injected at an interval of 10 to 50 days. When adjuvant was necessary, Sigma adjuvant system (S6322-1VL; Sigma-Aldrich) was used. Three days after the final immunization, splenocytes of the immunization host and P3U1 mouse myeloma cells were fused to prepare hybridomas. Screening for hybridomas producing anti-human PD-1 antibody was performed as follows. Briefly, the culture supernatant of each hybridoma was added to HEK293 cells which had been rendered to express human PD-1. After staining with R-phycoerythrin-labeled goat anti-mouse IgG(H+L) F(ab')2 fragment (115-116-146; Jackson ImmunoResearch) as secondary antibody, flow cytometry was performed for analysis. Finally selected hybridomas were cloned by limiting dilution, and cultured at high density in a cell line bioreactor (Wheaton). From the resultant culture supernatants, anti-human PD-1 antibodies were purified using Ab-Capture ExTra (P-003-10; Protenova).

### Antibody Sequencing

In order to specify the variable region sequences of anti-human PD-1 antibodies HM647 and HM698, frozen hybridomas were sent to BizComJapan, Inc. to use contract analysis services provided by Fusion Antibodies, Plc. Thus, the sequences were determined. Further, the variable region sequences of anti-human PD-1 antibodies HM266 and HM698 were determined by the method described in A Doenecke, E-L Winnacker and M Hallek Leukemia (1997) 11, 1787-1792. Briefly, total RNA was prepared from hybridoma cells, and cDNA of the variable region was PCR-amplified by 5'-RACE method using SMARTer^{™} RACE cDNA Amplification Kit (Clontech). Then, the cDNA sequence was determined. The resultant sequence was expressed as a recombinant human IgG1 antibody, whose specific binding to human PD-1 was confirmed. As regards the variable region sequence of HM698, the same sequence was obtained from either of the methods.

### Preparation and Purification of Recombinant Antibodies

A heavy chain expression vector was constructed using a DNA encoding a heavy chain sequence and an expression vector (pcDNA3.4, Thermo Fisher Scientific). Likewise, a light chain expression vector was constructed using a DNA encoding a light chain sequence and an expression vector (pcDNA3.4, Thermo Fisher Scientific). The two vectors were transfected into CHO cells or HEK293 cells by lipofection, and the transformed cells were cultured. Then, after removal of cells by centrifugation and filtration, the culture medium was collected. Antibodies were purified by a combination of affinity chromatography using Protein A or Capture Select kappaXI, column and gel filtration chromatography. Combinations of individual antibodies and purification methods are as described below:
Those purified by Protein A and then gel filtration: HM266-hIgG4-S228P, HM64 7 -hIgG 1-K322A, PD1AB6-hIgG1-K322A, PD1AB6-hIgG4-S228P, and PD1-17
Those purified by Capture Select kappaXL and then gel filtration: HM266-hIgG1-K322A, HM647-hIgG4-S228P, HM698-hIgG1-K322A, and HM698-hIgG4-S228P

### Activity Evaluation using Cell Lines

The activity of anti-human PD-1 antibody was evaluated as an effect on cytokine production caused by interaction between human PD-1-expressing T cells and antigen-presenting cells. Prepared as T cells were: a cell line obtained by treating DO11.10 T cell hybridoma cell line (kindly provided by Department of Immunology and Genomic Medicine, Graduate School of Medicine, Kyoto University) with Cas9 (Invitrogen) to knock-out mouse PD-1; and the knock-out cell line which was rendered to express human PD-1. Prepared as antigen-presenting cells were: a cell line obtained from IIA1.6 B cell line (kindly provided by Department of Immunology and Genomic Medicine, Graduate School of Medicine, Kyoto University) by knocking out mouse PD-L1; and the knock-out cell line which was rendered to express human PD-L1 or mouse FcyRIIB. For evaluating human PD-1 agonist activity, mouse FcyRIIB-expressing IIA1.6 cells were used; and for evaluating antagonist activity, human PD-L1-expressing IIA1.6 cells were used. Human PD-1-expressing DO11.10 T cell hybridomas and each IIA1.6 cells were suspended in a medium (RPMI1640 medium containing 10% fetal bovine serum), and were seeded in round bottom 96-well plates at 5 x 10⁴ and 1 x 10⁴ cells/well/50 µl, respectively. Anti-human PD-1 antibody was added to the plates to give a final concentration of 5, 0.5, 0.05 or 0.005 µg/ml at 50 µl/well.
Subsequently, OVA₃₂₃₋₃₃₉ peptide (Eurofins) was added as antigen to give a final concentration of 3 µg/ml at 50 µl/well. Eighteen hours later, IL-2 concentration in the culture supernatant was measured using mouse IL-2 DuoSet ELISA (R&D Systems).

In the evaluation of human PD-1 agonist activity, the cytokine suppression obtained by using human PD-L1-expressing IIA1.6 cells was taken as positive control; and the result obtained by using DO11.10 T cell hybridomas not rendered to express human PD-1 was taken as negative control. In the evaluation of human PD-1 antagonist activity, anti-human PD-1 antibody (clone: EH12.2H7) with known antagonist activity was used.

### Activity Evaluation on Human T Cells

The activity of anti-human PD-1 antibody against actual human T cells was evaluated. Specifically, human CD4⁺ T cells (LONZA) isolated from peripheral blood by negative selection were seeded in 24 well-plates pre-coated with anti-CD3 antibody (clone: OKT3, BioLegend) at 1 x 10⁶ cells/well/1 ml. Cells were activated by stimulating for 3 days to induce the expression of PD-1. As antigen-presenting cells, THP-1 human monocyte-derived cell line (ATCC) having forced expression of human FcyRIIB by electroporation were prepared. The resultant THP-1 cells were suspended in 500 µg/ml mitomycin C-added medium at 1 x 10⁷ cells/ml, incubated at 37°C for 2 hours to thereby terminate proliferation, and then used as antigen-presenting cells. Activated human CD4⁺ T cells and mitomycin-treated THP-1 cells were suspended in a medium and seeded in round bottom 96-well plates at 5 x 10⁴ and 2.5 x 10⁴ cells/well/50 µl, respectively. Anti-human PD-1 antibody was added to the plates at 50 µl/well to give a final concentration of 5, 0.5, 0.05 or 0.005 µg/ml. Subsequently, Cytostim (Miltenyi Biotec) was added as antigen at 50 µl/well to give a final concentration of 0.2 µl/well. Eighteen hours later, IL-2 concentration in the culture supernatant was measured using ELISA MAX Standard Set Human (BioLegend).

### Collection of Naive Helper T Cells (CD4⁺ CD62L⁺)

The spleen was collected from DO11.10 mouse (OVA₃₂₃₋₃₃₉ peptide-specific T cell receptor transgenic mouse; The Jackson Laboratory). Cell suspension was prepared and hemolysis was carried out. The resultant cells were stained with FITC-labeled antibodies to CD8, CD19, CD49b and I-A/I-E. After washing, magnetic bead-labeled anti-FITC antibody was bound to the cells, and CD4⁺ T cell fraction was fractionated by magnetic separation. This CD4⁺ T cell fraction was stained with PerCp-Cy5.5-labeled anti-CD4 antibody and APC-labeled anti-CD62L antibody, and CD4⁺ CD62L⁺ cells were collected by FACS to thereby obtain naive helper T cells. The thus obtained naive helper T cells were stimulated with Dynabeads mouse T activator (Invitrogen) for 24 hours in order to render the cells to express human PD-1.

### Forced Expression of Human PD-1 by Retrovirus Infection

Retrovirus supernatant was prepared by transfecting Plat-E cells with MSCV-hPD-1-IRES-Thy1.1. The retrovirus supernatant was added to RetroNectin^{™}-coated culture plates and centrifuged at 2500 rpm for 2 hours at 32°C. The centrifuged plates were washed with PBS, and then the CD4⁺ T cells stimulated as described above were added thereto and centrifuged at 2000 rpm for 10 minutes at 32°C. This operation for retrovirus infection was repeated twice with an interval of 24 hours. Thus, forced expression of human PD-1 was performed.

### Preparation of Antigen-Presenting Cells

11A1.6-mFcγRIIb(+) PD-L1(-) cells and 11A1.6-hPD-L1(+) cells were prepared respectively to give a concentration of 2 x 10⁷ cells/ml. An equal volume of mitomycin C (1 mg/mL) was added to each group of the cells, which were then treated at 37°C for 2 hours. The resultant cells were washed with PBS 3 times and used as antigen-presenting cells.

### Induction to Th1/Th2 Cells

To CD4⁺ T cells (1 x 10⁶ cells) rendered to express human PD-1, antigen-presenting cells (1 x 10⁶ cells) as well as OVA₃₂₃₋₃₃₉ peptide (final 5 µg/ml) and anti-human PD-1 antibody (final 5 µg/ml) were added in culture plates. For induction to Th1 cells, IL-2 (final 2 ng/ml; Peprotech), IL-12 (final 1 ng/mL; Peprotech), IFN-γ (final 1 ng/mL; Peprotech), and anti-IL-4 neutralizing antibody (final 5 µg/ml; clone 11B11, BioXcell) were added to give the indicated final concentrations. For induction of Th2 cells, IL-2 (final 2 ng/ml), IL-4 (final 1 ng/mL; Peprotech), anti-IFN-γ neutralizing antibody (final 5 µg/ml; clone XMG1.2, BioXcell), and anti-IL-12 neutralizing antibody (final 5 µg/mL; clone C17.8, BioXcell) were added to give the indicated final concentrations. Culture was performed in 12-well plates, and the volume of culture medium was 2 ml.

Seventy-two hours after the start of stimulation, one-half of the culture supernatant was discarded. Then, antigen-presenting cells (1 x 10⁶ cells) treated with mitomycin C as described above, OVA₃₂₃₋₃₃₉ peptide (final 5 µg/ml), anti-human PD-1 antibody (final 5 µg/ml), and Th1 and Th2 cytokine/neutralizing antibody cocktail were added thereto to make a culture medium having a final volume of 2 ml. FACS analysis was performed 96 hours after the start of stimulation.

### FACS Analysis by Intracellular Cytokine Staining

Th1/Th2 cells induced as described above were counted and seeded in preliminarily provided anti-CD3 antibody (2 µg/ml)-immobilized flat bottom 96-well plates at 2.5 x 10⁵ cells/well. After 4-hour culture at 37°C, brefeldin A (10 µg/ml) was added thereto and the cells were cultured further for 2 hours. Subsequently, the cells were collected from the plates. Fc receptors were blocked with anti-mouse CD16/32 antibody. Cell surfaces were stained with PerCp-Cy5.5-labeled anti-mouse CD4 antibody and Fixable Viability Dye eFluor780, washed with PBS, and subsequently fixed with 4% paraformaldehyde for 15 minutes. The fixed cells were washed with PBS, subjected to cell membrane permeabilization for 10 minutes, re-washed with PBS, stained with FITC-labeled anti-IFN-γ antibody and PE-labeled anti-IL-4 antibody for 45 minutes, and then analyzed by FACS.

### Preparation of Human PD-1 Knock-In Mouse using Genome Editing

gRNA targeting 5'-GCCAGGGGCTCTGGGCATGT-3' (SEQ ID NO: 23) and a donor vector containing human PD-1 gene was microinj ected into C57BL/6N mouse-derived pronuclear stage fertilized egg together with Cas9 protein (Invitrogen). The resultant egg was transplanted into the oviduct of foster mouse. With respect to the resultant mice (F0), indel mice were selected and mated with wild-type mice to obtain F1 mice. Those F1 mice confirmed for gene transfer were further mated to thereby obtain homozygous mice. These homozygous mice were used in experiments as human PD-1 knock-in mice.

### Evaluation of Anti-PD-1 Agonist Antibody Action against Antigen-Specific Antibody Subclass

NP-OVA(4-hydroxy-3-nitrophenylacetyl hapten-conjugated ovalbumin) was coprecipitated with alum adjuvant. A 100 µg aliquot was administered intraperitoneally to hPD-1 knock-in mice. Simultaneously, 500 µg of anti-human PD-1 antibody was intraperitoneally administered to the mice (Day 0). Further, the same dose of anti-human PD-1 antibody was administered intraperitoneally after 3 days and 7 days. Blood samples were collected from the orbit after 10 days, and plasma fraction was collected from peripheral blood by centrifugation. IgG1 and IgG2c antibody titers against the hapten in the plasma were measured by ELISA.

### Evaluation of PD-1 Agonist using House Dust Mite Extract (HDM)-Induced Allergy Model

HDM (D. Pteronyssinus; Greer) (total protein: 400 ng; 10 ng in terms of Derp1) was administered intraperitoneally to human PD-1 knock-in mouse. Simultaneously, 500 µg of anti-human PD-1 antibody was administered intraperitoneally (Day 0). Further, the same dose of anti-human PD-1 antibody was administered intraperitoneally after 3 days, 7 days and 10 days. In experiments under therapeutic regimen, anti-human PD-1 antibody was administered only once after 10 days. From 7 days after intraperitoneal administration of HDM, HDM (total protein 25 µg/25 µl) was administered intranasally under anesthesia. This intranasal administration was performed for 8 consecutive days. Four hours after final intranasal administration, mice were euthanized after collecting blood samples; and bronchoalveolar lavage fluid and the lung were collected. After centrifuging the bronchoalveolar lavage fluid, mononuclear cells were counted. The lung was subjected to enzyme treatment and density gradient centrifugation to fractionate mononuclear cells, which were then counted. The numbers of CD4⁺ T cells, CD8⁺ T cells, γδ TCR⁺ cells, eosinophils (CD11c⁻ SiglecF⁺), neutrophils (CD11c⁻ SiglecF⁻ Ly-6G⁺), alveolar macrophages (CD11c+ SiglecF+) and the like were determined by FACS analysis. Intracellular cytokines in bronchoalveolar lavage fluid-derived mononuclear cells and lung-derived mononuclear cells were stained by the above-described method. Blood concentration of HDM specific IgE was measured by ELISA (mouse serum anti-HDM IgE antibody assay kit; Chondrex).

### Evaluation of PD-1 Agonist using MC903-Induced Atopic Dermatitis Model

MC903 was used in the form of ethanol solution. MC903 at 5 nmol/10 µl was applied to both auricles of human PD-1 knock-in mice. Simultaneously, 500 µg of anti-human PD-1 antibody was administered intraperitoneally (Day 0). The same doses of MC903 and anti-human PD-1 antibody were respectively swabbed and administered every 3 days from Day 0 to Day 27. In experiments under therapeutic regimen, administration of anti-human PD-1 antibody was started from Day 12. During the experiment, the thickening of auricles was measured every 3 days. At Day 29, the number of times of scratching behavior in 10 minutes was visually counted. Blood samples were collected at Days 9, 15, 24 and 30. Plasma IgE concentrations were determined by ELISA (ELISAMAX Standard Set Mouse IgE; BioLegend). At Day 30, mice were euthanized and auricles were collected. The auricles were shredded and treated with enzymes to collect immune cells. The numbers of eosinophils (CD45⁺ CD11b⁺ SiglecF⁺) and the like infiltrating into the auricle tissue were determined by FACS analysis.

### Results and Discussion

In order to find out functional anti-PD-1 antibodies, the present inventors used an established cell line DO11.10 T cell hybridoma (Fig. 1). Since the activity of PD-1 signaling appears as suppression of T cell activation, necessary conditions for this experimental system are that sufficient T cell activation occurs and that PD-1- dependent immunosuppression is sure to occur. Although these conditions are feasible with a combination of primary cultured T cells and antigen-presenting cells, PD-1 protein is yet to be expressed in T cells before activation and, therefore, it is not possible to examine the effect on PD-1 signaling until after stimulation for activation has been performed. Since cell populations obtained by primary culture are not homogeneous in a variety of ways, they lack consistency between experiments. Moreover, with inevitable differences between individuals, the use of primary cultured cells is inconvenient in terms of consistency and reproducibility of the experimental system. Hence, for the purpose of screening antibodies, the present inventors have chosen an experimental system with an established cell line which is excellent in those two points.

DO11.10 T cell hybridoma is a T cell hybridoma cell line, which was originated from CD4⁺ T cells of DO11.10 mouse expressing MHC class II (I-A^{d})-restricted T cell receptor that recognizes an ovalbumin-derived peptide (OVA₃₂₃₋₃₃₉). DO11.10 T cell hybridoma produces IL-2 in response to the antigenic peptide presented on MHC class II (I-A^{d}) expressed on a B cell lymphoma cell line, IIA1.6 cells (Fig. 1A). Since this means that T cell activation in this combination of cells is inducible by antigen recognition, this experimental system has a substantial advantage of reproducing physiological immune activation scheme, which is not available in experimental systems using non-specific T cell activators. Furthermore, the modification of the cells such as gene knock-out and forced expression can be easily done. Therefore, an experimental system for examining the function of anti-human PD-1 antibody can be made by rendering mouse PD-1-deficient DO11.10 T cell hybridoma cells to express human PD-1. DO11.10 T cell hybridoma cells expressing human PD-1 clearly reduced IL-2 production upon antigen stimulation by PD-L1-expressing IIA1.6 cells, and this change was confirmed to be a PD-1-specific response (Fig. 1B). PD-1 and PD-L1 on the cell membrane should have a native protein conformation, which is also an advantage of this experimental system over artificial experimental systems using solubilized membrane proteins. Indeed, addition of anti-human PD-1 antibody (EH12.2H7), an established blocking antibody, to this experimental system reversed the PD-L1-dependent suppression of IL-2, indicating that this experimental system can reliably detect blocking antibodies (Fig. 1C). The present inventors have concluded that this experimental system using cellular interaction is a highly suitable combination for observing PD-1-mediated T cell suppression and conducted further experiments based on this system.

The evaluation of anti-PD-1 agonist antibodies having immunosuppressive activity should be conducted in the absence of PD-L1-dependent immunosuppression. To this end, a system different from the one for detecting a blocking antibody was provided. This system involves a combination of DO11.10 T cell hybridoma rendered to express human PD-1 and IIA1.6 cells rendered to lack PD-L1 but express FcyRIIB. Using two systems, one for detecting blocking antibodies and the other for detecting agonist antibodies, the present inventors performed screening of anti-human PD-1 monoclonal antibodies. The tested monoclonal antibodies were prepared from human PD-1-immunized mice. As a result, a number of anti-human PD-1 monoclonal antibodies with various degrees of activity were obtained (Fig. 2). Furthermore, antibodies with agonist activity were also found in commercially available anti-human PD-1 monoclonal antibodies such as MIH4 and J116. Comparison of the agonistic activity of these antibodies revealed that HM266, HM647, HM698 and MIH4 are especially favorable in agonist activity (Fig. 3).

In addition to the foregoing examination with mouse cells, these anti-PD-1 agonist antibodies were also confirmed to exhibit an immunosuppressive action on human T cells. IL-2 production from human peripheral blood-derived CD4⁺ T cells was remarkably suppressed by HM266 (Fig. 4).

The functions of CD4⁺ helper T cells are diverse and such various functions are covered by different subsets of helper T cells which play respective roles specialized for individual immune functions. As typical examples, cell subsets such as Th1, Th2, Th17, Treg, etc. with different roles are known. For example, Th1 cells promote cellular immunity, which mainly involves direct cytotoxicity by CD8⁺ cells and NK cells, and Th2 cells promote class switching to IgE by enhancement of humoral immunity and promote allergic response by activation of eosinophils. Thus, helper T cell subsets are specialized for greatly different immune functions. Respective helper T cell subsets produce greatly different cytokines. While IFN-γ is characteristic of Th1 cells, IL-4, IL-5 and IL-13 are characteristic of Th2 cells, and IL-17 is characteristic of Th17 cells. Each subset has a characteristic pattern of cytokine production. Helper T cell subsets regulate different immune functions through these cytokines.

Cytokine production from helper T cell subsets is also one of the functions under the control of PD-1. A number of researchers have reported increases of cytokine production when PD-1 or PD-1 ligand (PD-L1 or PD-L2) is blocked. In many cases, there is no difference depending on the type of cytokine. It is reported that the blockade of PD-1 signaling increased all of IFN-γ, TNF-α, IL-4, IL-10 and IL-13 when peripheral blood lymphocytes from patients with various allergies were re-stimulated with relevant causative allergens^{[1]}. Researchers who point out differences depending on the type of cytokine report that production of IFN-γ is larger than the production of IL-4, IL-5, IL-13, etc. at the time of PD-1 blocking, with Th1 cells becoming dominant over Th2 cells^{[2-5]}. When PD-1 was actively stimulated with PD-L1, PD-L2 or the like, cytokine production from those T cells which are expressing the PD-1 is suppressed. However, even in this case, no remarkable difference is observed between Th1-type and Th2-type cytokines^{[6-8]}. In experiments using PD1-17 (Wyeth) as hPD-1 agonist antibody, it was also reported that IFN-γ, IL-2, IL-10 and IL-13 are suppressed to the similar extent^{[9]}. These experimental results refer to the function of PD-1/PD-1 ligand against cytokine-producing capacity of Th1 cells, Th2 cells, or other Th subsets which were already present in the samples. However, earlier than this stage, in order for such Th1 and Th2 cells to exist, there should have been processes that brought about their development. For elucidating the effect of PD-1 stimulation at the stage of Th1/Th2 development, examination from a different approach must be made.

Helper T cell subsets such as Th1 and Th2 are considered to be induced from naive helper T cells of the same origin. For example, the same naive helper T cells can become Th1 cells when activated in the presence of IL-12 and become Th2 cells when activated in the presence of IL-4. This process is called functional differentiation. This fact means that naive helper T cells could differentiate into either Th1 or Th2 cell-dominant population depending on the environment at the time of activation, and that whether relative immune responses are directed toward inflammations caused by cytotoxic immune responses or toward allergic inflammatory responses caused mainly by antibodies and eosinophils is partly determined by the functional differentiation of naive helper T cells. Therefore, if the balance between Th1 and Th2 existence was changed by PD-1 stimulation, immune responses would not only be suppressed in quantity but also be changed in quality.

Then, the present inventors have examined the effect of PD-1 agonist stimulation on functional differentiation of CD4⁺ helper T cells. Briefly, naive helper T cells from DO11.10 mouse were rendered to express hPD-1 using retrovirus. When the resultant naïve T cells were induced to differentiate into Th1 and Th2 under different cytokine conditions, anti-human PD-1 agonist antibody (HM266, HM647 or 1116) was added. As a result, the addition of any of the anti-human PD-1 agonist antibodies greatly decreased the proportion of IL-4-producing Th2 cells (Fig. 5). In contrast, differentiation to IFN-γ-producing Th1 cells showed resistance against the agonist antibodies. When control antibody was used, no such suppression of differentiation to Th2 cells was observed. In PD-1 stimulation with PD-L1 expressing cells, the proportion of Th2 cells also decreased greatly whereas the effect on Th1 cell induction was relatively mild, showing a similar tendency as seen with anti-human PD-1 agonist antibodies.

The results described so far indicate consistently that, at the time of functional differentiation of helper T cells, sensitivity to suppressive action of PD-1 agonist differs between Th subsets and that differentiation to Th2 is highly sensitive to PD-1 signaling.

Very few studies have so far discussed the role of PD-1 in functional differentiation to Th1/Th2 cells. Such studies were conducted using immobilized PD-L1 on plastic surfaces and the outcome was that differentiation to Th2 was not inhibited, though suppression of Th1 (and Th17) was observed^{[10, 11]}. Unlike these articles, the experimental system of the present inventors reproduces antigen-specific activation through interaction between T cells and antigen-presenting cells, providing the result showing that functional differentiation to Th2 has particularly high sensitivity to PD-1 stimulation.

Suppression of Th2 type immune response by PD-1 agonist was also shown in *in vivo* experiments. Antibodies induced from B cells in response to antigen progressively mature through mechanism of class switching and the like, receiving help from T cells. It is known that class switching is induced specifically, e.g., class switching to IgG2a and IgG2c by the action of Th1 cells and class switching to IgG1 or IgE by the action of Th2 cells. When human PD-1 knock-in mice were immunized with NP-OVA and NP-specific IgG was analyzed, its overall production was suppressed by the administration of anti-PD-1 agonist antibody. However, when the present inventors focused on IgG subclasses, the suppressive action of the agonist antibody was more dominant on IgG1 than on IgG2c, and the ratio of IgG1/IgG2c was decreased (Fig. 6). These results suggest that PD-1 agonists may be able to change Th1/Th2 balance in immune responses *in vivo* and selectively suppress Th2-type immunity.

In view of the above-described finding that functional differentiation to Th2 cells is strongly suppressed by PD-1 agonist stimulation, allergic diseases are thought to be one of the diseases for which the efficacy of PD-1 agonist can be expected. Allergic diseases, including asthma, atopic dermatitis, pollinosis and drug/food allergy, are diseases having a great number of patients. Recently, the number of patients with these diseases tends to increase sharply on a global scale. An effective treatment for these diseases is strongly demanded. These allergic diseases are immune responses induced by Th2-type cytokine production and their characteristic changes are eosinophils-dominant inflammation and increase of blood IgE concentration. The increased IgE enhances activation of mast cells to expand inflammation. Although the direct cause of the increase of IgE antibody concentration is an increase of B cells that produce IgE antibody, it is the action of cytokines such as IL-4 and IL-13 released from Th2 cells that induces class switching of antibodies to IgE. Th2 cells are capable of inducing activation of eosinophils by producing IL-5 in addition to these cytokines. In other words, Th2 cells increase IgE production in B cells by means of IL-4 and IL-13 and, simultaneously, Th2 cells use IL-5 to enhance eosinophil-dominant inflammation. Therefore, it is considered that increase of Th2 cells plays a central role in the pathology of allergic diseases. Accordingly, if the balance of functional differentiation of helper T cells is considerably biased toward a Th2-dominant state, this would lead to the onset of allergic diseases not far off. From these backgrounds, it is suggested that suppression of excessive Th2-type immune response is effective for the treatment of allergic diseases.

According to the present inventors' new finding that PD-1 agonist has a particularly strong suppressive action against functional differentiation to Th2 cells, PD-1 agonist is expected to be effective in suppressing the onset of allergic diseases in which Th2 cells are playing a central role and also for treating such diseases. Accordingly, the effect of PD-1 agonist on allergic asthma was examined using an experimental animal model of allergic asthma in which the pathological condition was induced by causing mice to inhale house dust mite antigen (HDM). This experiment was conducted according to a schedule in which mice were sensitized with HDM and allowed to inhale the same antigen daily from 7 days after the sensitization. To examine the efficacy of anti-PD-1 agonist antibody on allergic asthma, administration of anti-PD-1 agonist antibody was started at the time of sensitization. Subsequently, administration of anti-PD-1 agonist antibody was continued at a frequency of twice a week, and 2 weeks after the start of sensitization, mice were euthanized for analysis. Administration of J116 significantly reduced infiltration of eosinophils into the alveoli (Fig. 7). Correspondingly, analysis of CD4⁺ T cells infiltrating into the alveoli showed decreases in the numbers of IL-5 and IL-13-producing cells (Fig. 8).

When J116 was replaced by HM266 having a stronger agonist activity, anti-inflammatory effect on allergic inflammation was also enhanced. HM266 suppressed the infiltration of eosinophils and CD4⁺ T cells into the alveoli, and the CD4⁺ T cells which produced IL-4, IL-5 or IL-13 decreased across the board (Figs. 9 and 10). Corresponding to the decrease of Th2 cells, blood concentration of HDM-specific IgE decreased greatly, indicating that Th2-type immunity was suppressed (Fig. 10). Furthermore, the present inventors validated the efficacy of anti-PD-1 agonist antibody under a therapeutic regimen. In this case, HM266 was administered only once on day 3 after the start of daily inhalation of the antigen, and infiltration of eosinophils and Th2 type cytokine-producing CD4⁺ T cells was significantly suppressed by HM266 (Figs. 9 and 10). These results indicate that PD-1 agonist is useful as a preventive and a therapeutic for allergic inflammations.

The present inventors examined anti-inflammatory effect of anti-PD-1 agonist antibody on the induction of atopic dermatitis as a different sort of type I allergic disease model. Allergic dermatitis was induced in human PD-1 knock-in mice by applying MC903 on the auricle. Simultaneous administration of HM266 significantly suppressed swelling in the auricle (Fig. 11). Suppression of allergic inflammatory responses is notable as indicated by the marked decrease of blood IgE concentration, the number of eosinophils infiltrating into the auricle tissue, and the reduced scratching behavior of mice. Furthermore, to examine the effect of its therapeutic administration, the present inventors started HM266 administration after the swelling in the auricle was induced (from Day 12). In this therapeutic regimen, HM266 markedly reduced IgE levels and eosinophils infiltration (Fig. 11). These results suggest the possibility that PD-1 agonist would be useful for treating type I allergy and other Th2 type immunity-mediated diseases in general.

Although there have been previous studies in which PD-1, PD-L1 or PD-L2 was inhibited in animal models of asthma, the results are various and even include entirely opposite reports. Therefore, the role of PD-1 in the pathology of asthma is highly debatable. There is an article reporting that allergic inflammation worsened when PD-1/PD-L1 were blocked. However, the authors of this article do not recognize enhancement of Th2 and conclude that the worsening was caused by enhancement of Th17 type immunity^{[11]}. As opposed to this study, some researchers report that the blocking of PD-1/PD-L1 did not worsen the inflammation. In these models, the blocking of PD-L2 enhanced inflammation^{[12-14]} and resulted in Th2-dominant immune status as demonstrated by increases of IL-5 and IL-13 and a decrease of IFN-γ^{[12]}. However, in the same study, the blocking of PD-1 was not shown to have any effect on inflammation, and the authors of this paper concluded that the change caused by PD-L2 blocking was an event not dependent on PD-1. For studies on asthma and PD-L2, PD-L2-Fc was also administered in asthma model. In that study, the addition of PD-L2-Fc was suggested to suppress cytokine production from T cells *in vitro.* However, the results of *in vivo* treatment with PD-L2-Fc was inconsistent with the *in vitro* result since PD-L2-Fc increased IL-5, IL-13 and IgE in vivo along with the exacerbation of inflammation ^{[6]}. This result is hardly compatible with the exacerbation of inflammation by PD-L2 blockade in other studies.

The existing reports may be summarized as follows. Although it has been known that PD-1 stimulation suppresses various cytokines, there has been no report that PD-1 stimulation selectively suppresses Th2 cells. In regard to the role of PD-1 agonist in functional differentiation of CD4⁺ T cells, no study has ever suggested that Th2 differentiation is particularly susceptible to PD-1 agonists. Furthermore, the current understandings for the role of PD-1 in asthma models still remains controversial as studies have presented contradictory results. Among these, there has been no reports demonstrating that suppression of not only Th2 type immunity but also asthma and allergic dermatitis is induced by PD-1 stimulation.

To cope with such circumstances, the studies of the present inventors have revealed that sensitivity to PD-1 stimulation differs between Th1 and Th2 cells and that functional differentiation to Th2 cells is strongly inhibited by PD-1 stimulation. The present inventors have also demonstrated that by using PD-1 agonist, in particular anti-PD-1 agonist antibody, it is possible to change the immune balance between Th1 and Th2 to thereby regulate Th2-mediated biological response. Indeed, the present inventors have made it clear that, even *in vivo,* establishment of Th2 type immunity in mouse is inhibited and allergic inflammation in the tissue is markedly suppressed by administration of anti-PD-1 agonist antibody. The results of the present invention indicate that anti-PD-1 agonist antibody is capable of correcting Th2-biased immune balance and is useful for preventing and treating Th2-mediated diseases, especially type I allergy and eosinophilic disorders.

### References

1. Rosskopf S, Jahn-Schmid B, Schmetterer KG, Zlabinger GJ, Steinberger P. PD-1 has a unique capacity to inhibit allergen-specific human CD4+ T cell responses. Sci. Rep. 8:13543 (2018)
2. Rosenblatt J, Glotzbecker B, Mills H, Vasir B, Tzachanis D, Levine JD, Joyce RM, Wellenstein K, Keefe W, Schickler M, Rotem-Yehudar R, Kufe D, Avigan D. PD-1 blockade by CT-011, anti-PD-1 antibody, enhances ex vivo T-cell responses to autologous dendritic cell/myeloma fusion vaccine. J. Immunother. 34:409-18 (2011).
3. Dulos J, Carven GJ, van Boxtel SJ, Evers S, Driessen-Engels LJ, Hobo W, Gorecka MA, de Haan AF, Mulders P, Punt CJ, Jacobs JF, Schalken JA, Oosterwijk E, van Eenennaam H, Boots AM. PD-1 blockade augments Th1 and Th17 and suppresses Th2 responses in peripheral blood from patients with prostate and advanced melanoma cancer. J. Immunother. 35:169-78 (2012).
4. Wang S, Zhu X, Xu Y, Zhang D, Li Y,Tao Y, Piao H, Li D, Du M. Programmed cell death-1 (PD-1) and T-cell immunoglobulin mucin-3 (Tim-3) regulate CD4+ T cells to induce Type 2 helper T cell (Th2) bias at the maternal-fetal interface. Hum. Reprod. 31:700-11 (2016).
5. Rajamanickam A, Munisankar S, Dolla C, Nutman TB, Babu S. Cytotoxic T-Lymphocyte-Associated Antigen 4 (CTLA-4) and Programmed Death 1 (PD-1) mediated regulation of mono- and dual functional CD4+ and CD8+ T-cell responses in a chronic helminth infection. Infect. Immun. 87:e00469-19 (2019).
6. Oflazoglu E, Swart DA, Anders-Bartholo P, Jessup HK, Norment AM, Lawrence WA, Brasel K, Tocker JE, Horan T, Welcher AA, Fitzpatrick DR. Paradoxical role of programmed death-1 ligand 2 in Th2 immune responses in vitro and in a mouse asthma model in vivo. Eur. J. Immunol. 34:3326-36 (2004).
7. Zhu B, Guleria I, Khosroshahi A, Chitnis T, Imitola J, Azuma M, Yagita H, Sayegh MH, Khoury SJ. Differential role of programmed death-ligand 1 [corrected] and programmed death-ligand 2 [corrected] in regulating the susceptibility and chronic progression of experimental autoimmune encephalomyelitis. J. Immunol. 176:3480-9 (2006).
8. Kubo S, Yamada T, Osawa Y, Ito Y, Narita N, Fujieda S. Cytosine-phosphate-guanosine-DNA induces CD274 expression in human B cells and suppresses T helper type 2 cytokine production in pollen antigen-stimulated CD4-positive cells. Clin. Exp. Immunol. 169:1-9 (2012).
9. Chemnitz JM, Parry RV, Nichols KE, June CH, Riley JL. SHP-1 and SHP-2 associate with immunoreceptor tyrosine-based switch motif of programmed death 1 upon primary human T cell stimulation,but only receptor ligation prevents T cell activation. J. Immunol. 173:945-54 (2004).
10. Herold M, Posevitz V, Chudyka D, Hucke S, Groβ C, Kurth F, Leder C, Loser K, Kurts C, Knolle P, Klotz L, Wiendl H. B7-H1 Selectively Controls TH17 Differentiation and Central Nervous System Autoimmunity via a Novel Non-PD-1-Mediated Pathway. J. Immunol. 195:3584-95 (2015).
11. McAlees JW, Lajoie S, Dienger K, Sproles AA, Richgels PK, Yang Y, Khodoun M, Azuma M, Yagita H, Fulkerson PC, Wills-Karp M, Lewkowich IP. Differential control of CD4(+) T-cell subsets by the PD-1/PD-L1 axis in a mouse model of allergic asthma. Eur. J. Immunol. 45:1019-29 (2015).
12. Matsumoto K, Inoue H, Nakano T, Tsuda M, Yoshiura Y, Fukuyama S, Tsushima F, Hoshino T, Aizawa H, Akiba H, Pardoll D, Hara N, Yagita H, Azuma M, Nakanishi Y B7-DC regulates asthmatic response by an IFN-gamma-dependent mechanism. J. Immunol. 172:2530-41 (2004).
13. Akbari O, Stock P, Singh AK, Lombardi V, Lee WL, Freeman GJ, Sharpe AH, Umetsu DT, Dekruyff RH. PD-L1 and PD-L2 modulate airway inflammation and iNKT-cell-dependent airway hyperreactivity in opposing directions. Mucosal Immunol. 3:81-91 (2010).
14. Lewkowich IP, Lajoie S, Stoffers SL, Suzuki Y, Richgels PK, Dienger K, Sproles AA, Yagita H, Hamid Q, Wills-Karp M. PD-L2 modulates asthma severity by directly decreasing dendritic cell IL-12 production. Mucosal Immunol. 6:728-39 (2013).

All publications, patents and patent applications cited herein are incorporated herein by reference in their entirety.

### INDUSTRIAL APPLICABILITY

The present invention is applicable to treatment and/or prevention of Th2-mediated diseases such as type I allergy or eosinophilic diseases.

### SEQUENCE LISTING FREE TEXT

Clone name: HM-266
VH Amino Acid Sequence 
QVQLQQSGPELVKPGASVKMSCKASGYTFTSYYIQWVKQRPGQGLEWIGWIYPGDG
SSKYNEKFKGKTTLTADKSSSTAYMLLSSLTSEDSGIYFCASYYGSSFDYWGQGTTLT
VSS (SEQ ID NO: 1) Origin: Mus musculus
VH DNA Sequence 
Caggtccagctgcagcagtctggacctgagctggtgaagcctggggcttcagtgaagatgtcctgcaaggcttctggctacaccttca
caagctactatatacagtgggtgaagcagaggcctggacagggacttgagtggattggatggatttatcctggagatggtagtagcaa
gtacaatgagaagttcaagggcaagaccacactgactgcagacaaatcctccagcacagcctacatgttgctcagcagcctgacctct
 
gaggactctgggatctatttctgtgcaagttactacggtagtagttttgactactggggccaaggcaccactctcacagtctcctca
(SEQ ID NO: 2) Origin: Mus musculus
VL Amino Acid Sequence 
DIQMTQTTSSLSASLGDRVTISCRASQDISNYLNWYQQKPDGTVKFLIYYSSRLHSGV
PSRFSGSGSGTDYSLTISNLEQEDIATYFCQQGSTLPFTFGGGTKLEIK (SEQ ID NO: 3)
Origin: Mus musculus
VL DNA Sequence 
Gatatccagatgacacagactacatcctccctgtctgcctctctgggagacagagtcaccatcagttgcagggcaagtcaggacatta
gcaattatttaaactggtatcagcagaaaccagatggaactgttaaattcctaatatactactcatcaagattacactcaggagtcccatca
aggttcagtggcagtgggtctggaacagattattctctcaccattagcaacctggagcaggaagatattgccacttacttttgccagcag
ggtagtacgcttccgttcacgttcggaggggggaccaagctggaaataaaa (SEQ ID NO: 4) Origin: Mus musculus
Clone name: HM-647
VH Amino Acid Sequence 
EVLLQQSGPELVKPGASVKITCKASGYTFTDYNMDWVKQSHGKSLEWIGDIDPNNG
GTVYNQKFKDKASLTVDKSSSTGYMELRSLTSEDTAVYYCARWRSAMDYWGQGTS
VTVSS (SEQ ID NO: 5) Origin: Mus musculus
VH DNA Sequence 
Gaggtcctgctgcaacagtctggacctgagctggtgaagcctggggcttcagtgaagataacctgcaaggcttctggatacacattca
ctgactacaacatggactgggtgaagcagagccatggaaagagccttgagtggattggagatattgatcctaacaatggtggtactgtc
tacaaccagaagttcaaggacaaggcctcattgactgtagacaagtcctccagcacaggctacatggagctccgcagcctgacatctg
aggacactgcggtctattactgtgcaagatggcggagtgctatggactactggggtcaaggaacctcagtcaccgtctcctca (SEQ
ID NO: 6) Origin: Mus musculus
VL Amino Acid Sequence 
DIQMTQPTSSLSASLGDRVTISCRASQDISNYLNWYQQKPDGTVKLLISYTSRLHSGV
PSRFSGSGSGTDYSLTISNLEQVDIATYFCQQYSTLPWTFGGGSKLEIK (SEQ ID NO: 7)
Origin: Mus musculus
VL DNA Sequence 
Gatatccagatgacacagcctacatcctccctgtctgcctctctgggagacagagtcaccatcagttgcagggcaagtcaggacatta
gcaattatttaaactggtatcaacagaaaccagatggaactgttaaactcctgatctcctacacatcaagattacactcaggagtcccatc
aaggttcagtggcagtgggtctggaacagattattctctcaccattagcaacctggagcaggtagatattgccacttacttttgccaacag
tatagtacgcttccgtggacgttcggtggaggctccaagctggaaatcaaa (SEQ ID NO: 8) Origin: Mus musculus
Clone name: HM-698
VH Amino Acid Sequence 
EVLLQQSGPELVKPGASVKIPCKASGYTFTDYNMDWVKQSHGKSLEWIGDIDPNNG
GTVYNQKFKGKASLTVDKSSSTAYMELRSLTSEDTAVYYCARWRSAMDYWGQGTS
VTVSS (SEQ ID NO: 9) Origin: Mus musculus
VH DNA Sequence 
Gaggtcctgctgcaacagtctggacctgaactagtgaagcctggggcttcagtgaagataccctgcaaggcttctggatacacattca
ctgactacaacatggactgggtgaagcagagccatggaaagagccttgagtggattggagatattgatcctaacaatggtggtactgtc
tacaaccagaagttcaagggcaaggcctcattgactgtagacaagtcctccagcacagcctacatggagctccgcagcctgacatctg
 
aggacactgcggtctattactgtgcaagatggcggagtgctatggactactggggtcaaggaacctcagtcaccgtctcctca (SEQ
ID NO: 10) Origin: Mus musculus
VL Amino Acid Sequence 
DIQMTQTTSSLSASLGDRVTISCRASQDISNYLNWYQQKPDGTVKLLIYYTSRLHSGV
PSRFSGSGSGTDYSLTISNLEQEDIATYFCQQYNTLPWTFGGGTKLEIK (SEQ ID NO:
11) Origin: Mus musculus
VL DNA Sequence 
Gatatccagatgacacagactacatcctccctgtctgcctctctgggagacagagtcaccatcagttgcagggcaagtcaggacatta
gcaattatttaaactggtatcagcagaaaccagatggaactgttaaactcctgatctactacacatcaagattacactcaggagtcccatc
aaggttcagtggcagtgggtctggaacagattattctctcaccattagcaacctggagcaagaagatattgccacttacttttgccaacag
tataatacgcttccgtggacgttcggtggaggcaccaaactggaaatcaaa (SEQ ID NO: 12) Origin: Mus
musculus
Mouse IgG1 CH
Amino Acid Sequence 
AKTTPPSVYPLAPGSAAQTNSMVTLGCLVKGYFPEPVTVTWNSGSLSSGVHTFPAVL
ESDLYTLSSSVTVPSSPRPSETVTCNVAHPASSTKVDKKIVPRDCGCKPCICTVPEVSS
VFIFPPKPKDVLTITLTPKVTCVVVDISKDDPEVQFSWFVDDVEVHTAQTQPREEQFN
STFRSVSELPIMHQDWLNGKEFKCRVNSAAFPAPIEKTISKTKGRPKAPQVYTIPPPKE
QMAKDKVSLTCMITDFFPEDITVEWQWNGQPAENYKNTQPIMNTNGSYFVYSKLNV
QKSNWEAGNTFTCSVLHEGLHNHHTEKSLSHSPGK (SEQ ID NO: 13) Origin: Mus
musculus
DNA Sequence 
Gccaaaacgacacccccatctgtctatccactggcccctggatctgctgcccaaactaactccatggtgaccctgggatgcctggtca
agggctatttccctgagccagtgacagtgacctggaactctggatccctgtccagcggtgtgcacaccttcccagctgtcctggagtct
gacctctacactctgagcagctcagtgactgtcccctccagccctcggcccagcgagaccgtcacctgcaacgttgcccacccggcc
agcagcaccaaggtggacaagaaaattgtgcccagggattgtggttgtaagccttgcatatgtacagtcccagaagtatcatctgtcttc
atcttccccccaaagcccaaggatgtgctcaccattactctgactcctaaggtcacgtgtgttgtggtagacatcagcaaggatgatccc
gaggtccagttcagctggtttgtagatgatgtggaggtgcacacagctcagacgcaaccccgggaggagcagttcaacagcactttcc
gctcagtcagtgaacttcccatcatgcaccaggactggctcaatggcaaggagttcaaatgcagggtcaacagtgcagctttccctgcc
cccatcgagaaaaccatctccaaaaccaaaggcagaccgaaggctccacaggtgtacaccattccacctcccaaggagcagatggc
caaggataaagtcagtctgacctgcatgataacagacttcttccctgaagacattactgtggagtggcagtggaatgggcagccagcg
gagaactacaagaacactcagcccatcatgaacacgaatggctcttacttcgtctacagcaagctcaatgtgcagaagagcaactggg
aggcaggaaatactttcacctgctctgtgttacatgagggcctgcacaaccaccatactgagaagagcctctcccactctcctggtaaa
(SEQ ID NO: 14) Origin: Mus musculus
Mouse Igk CL
Amino Acid Sequence 
RADAAPTVSIFPPSSEQLTSGGASVVCFLNNFYPKDINVKWKIDGSERQNGVLNSWT
DQDSKDSTYSMSSTLTLTKDEYERHNSYTCEATHKTSTSPIVKSFNRNEC (SEQ ID NO:
15) Origin: Mus musculus
DNA Sequence 
Cgggctgatgctgcaccaactgtatccatcttcccaccatccagtgagcagttaacatctggaggtgcctcagtcgtgtgcttcttgaac
aacttctaccccaaagacatcaatgtcaagtggaagattgatggcagtgaacgacaaaatggcgtcctgaacagttggactgatcagg
 
acagcaaagacagcacctacagcatgagcagcaccctcacgttgaccaaggacgagtatgaacgacataacagctatacctgtgag
gccactcacaagacatcaacttcacccattgtcaagagcttcaacaggaatgagtgt (SEQ ID NO: 16) Origin: Mus
musculus
Human IgG1-K322A CH Amino Acid Sequence 
ASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKT
KPREEQYNSTYRVVSVLTVLHQDWLNGKEYKCAVSNKALPAPIEKTISKAKGQPREP
QVYTLPPSRDELTKNQVSLTCLVKGFYPSDIAVEWESNGQPENNYKTTPPVLDSDGSF
FLYSKLTVDKSRWQQGNVFSCSVMHEALHNHYTQKSLSLSPGK* (SEQ ID NO: 17)
Origin: artificial
IgG1-K322A CH DNA Sequence 
Gcgtcgaccaagggcccctccgtgttccccctggccccctcctccaagtccacctccggcggcaccgccgccctgggctgcctggt
gaaggactacttccccgagcccgtgaccgtgtcctggaactccggcgccctgacctccggcgtgcacaccttccccgccgtgctcca
gtcctccggcctgtactccctgtcctccgtggtgaccgtgccctcctcctccctgggcacccagacctacatctgcaacgtgaaccaca
agccctccaacaccaaggtggacaagaaggtcgagcccaagtcctgcgacaagacccacacctgccccccctgccccgcccccga
gctgctgggcggcccctccgtgttcctgttcccccccaagcccaaggacaccctgatgatctccaggacccccgaggtgacctgcgt
ggtggtggacgtgtcccacgaggaccccgaggtgaagttcaactggtacgtggacggcgtggaggtgcacaacgccaagaccaag
cccagggaggagcagtacaactccacctacagggtggtgtccgtgctgaccgtgctgcaccaggactggctgaacggcaaggagt
acaagtgcgccgtgtccaacaaggccctgcccgcccccatcgagaagaccatctccaaggccaagggccagcccagggagcccc
aggtgtacaccctgcccccctccagggacgagctgaccaagaaccaggtgtccctgacctgcctggtgaagggcttctacccctccg
acatcgccgtggagtgggagtccaacggccagcccgagaacaactacaagaccaccccccccgtgctggactccgacggctccttc
ttcctgtactccaagctgaccgtggacaagtccaggtggcagcagggcaacgtgttctcctgctccgtgatgcacgaggccctgcaca
accactacacccagaagtccctgtccctgtcccccggcaagtga (SEQ ID NO: 18) Origin: artificial
IgG4-S228P CH Amino Acid Sequence 
ASTKGPSVFPLAPCSRSTSESTAALGCLVKDYFPEPVTVSWNSGALTSGVHTFPAVLQS
SGLYSLSSVVTVPSSSLGTKTYTCNVDHKPSNTKVDKRVESKYGPPCPPCPAPEFLGG
PSVFLFPPKPKDTLMISRTPEVTCVVVDVSQEDPEVQFNWYVDGVEVHNAKTKPREE
QFNSTYRVVSVLTVLHQDWLNGKEYKCKVSNKGLPSSIEKTISKAKGQPREPQVYTL
PPSQEEMTKNQVSLTCLVKGFYPSDIAVEWESNGQPENNYKTTPPVLDSDGSFFLYSR
LTVDKSRWQEGNVFSCSVMHEALHNHYTQKSLSLSPGR* (SEQ ID NO: 19) Origin:
artificial
IgG4-S228P CH DNA Sequence 
Gcctcaacgaagggtccttctgtctttcccttggcaccttgttctcggtccacaagcgagagcaccgcggctctgggctgcttggtgaa
agactacttccccgagcccgttacagtgtcctggaacagtggagctttgacttctggggtgcatactttcccagctgtgctgcagtctagc
ggactctatagccttagctcagtggtaactgtaccttcttcctcactcgggaccaaaacctacacatgcaatgtggaccacaaacccagt
aataccaaagtggacaaacgtgtcgagtccaaatatggcccgccctgtccaccttgtccagctccagaatttctgggaggacctagcgt
attcctgtttcctccgaaacctaaggatacgctcatgatttctcgcactcccgaagtgacatgtgttgtggtcgacgtgagtcaagaagat
ccagaggttcagttcaactggtatgtcgatggggttgaggtccacaatgccaagactaagcctagagaggagcagtttaactccacata
ccgagtcgtgtcagtgctgaccgtcctgcatcaggactggctcaatgggaaggagtacaaatgcaaagtctccaataagggcctgcc
aagtagcatagaaaagaccatttcaaaagcgaaaggccaaccccgggaaccccaggtgtataccctgccaccaagccaagaggag
atgaccaagaatcaggttagtctcacttgcctggtcaaaggattctatccctctgacatcgccgtcgaatgggagtccaatggccagcc
cgaaaacaactacaagaccacacctccagttctggacagcgacggtagtttcttcctgtactcacgcctgacagtggacaagagcaga
tggcaggaaggcaacgtattcagctgctccgtgatgcacgaagccctgcacaatcactacacacagaagagtttgagcctttcccctg
 gtagatga (SEQ ID NO: 20) Origin: artificial
Human Igk CL Amino Acid Sequence 

* (SEQ ID
NO: 21) Origin: Homo sapiens
CL DNA Sequence 
Cgaactgtagccgcaccaagcgtgttcatctttccgccatccgatgaacagctgaagtcaggcacagcgtcagttgtctgtctcctcaa
caacttctaccccagagaggccaaagtgcagtggaaagtggacaatgccctgcagtcaggcaattctcaggaatctgtgacagagca
ggactccaaagacagtacctatagcctgtctagcacactgacgctctctaaggccgactatgagaagcacaaggtctatgcctgtgaa
gtgacacatcaagggctgagcagtccagtcactaagagcttcaatcgtggggaatgctga (SEQ ID NO: 22) Origin:
Homo sapiens
gRNA(5'-GCCAGGGGCTCTGGGCATGT-3') (SEQ ID NO: 23) Origin: artificial
Amino Acid Sequence of Human PD-1 
MQIPQAPWPVVWAVLQLGWRPGWFLDSPDRPWNPPTFSPALLVVTEGDNATFTCSFS
NTSESFVLNWYRMSPSNQTDKLAAFPEDRSQPGQDCRFRVTQLPNGRDFHMSVVRA
RRNDSGTYLCGAISLAPKAQIKESLRAELRVTERRAEVPTAHPSPSPRPAGQFQTLVVG
VVGGLLGSLVLLVWVLAVICSRAARGTIGARRTGQPLKEDPSAVPVFSVDYGELDFQ
WREKTPEPPVPCVPEQTEYATIVFPSGMGTSSPARRGSADGPRSAQPLRPEDGHCSWP
L (SEQ ID NO: 24)
Amino Acid Sequence of Mouse PD-1

MWVRQVPWSFTWAVLQLSWQSGWLLEVPNGPWRSLTFYPAWLTVSEGANATFTCSL
SNWSEDLMLNWNRLSPSNQTEKQAAFCNGLSQPVQDARFQIIQLPNRHDFHMNILD
TRRNDSGIYLCGAISLHPKAKIEESPGAELVVTERILETSTRYPSPSPKPEGRFQGMVIG
IMSALVGIPVLLLLAWALAVFCSTSMSEARGAGSKDDTLKEEPSAAPVPSVAYEELDF
QGREKTPELPTACVHTEYATIVFTEGLGASAMGRRGSADGLQGPRPPRHEDGHCSWP
L (SEQ ID NO: 25)

## Claims

1. A pharmaceutical composition for treating or preventing Th2-mediated diseases, the composition comprising an effective amount of a PD-1 agonist.

2. The pharmaceutical composition of claim 1, wherein the PD-1 agonist is an anti-PD-1 agonist antibody or a functional fragment thereof.

3. The pharmaceutical composition of claim 1 or 2, wherein the Th2-mediated disease is type I allergy.

4. The pharmaceutical composition of claim 1 or 2, wherein the Th2-mediated disease is an eosinophilic disease.

5. The pharmaceutical composition of claim 1 or 2, wherein the Th2-mediated disease is a disease selected from the group consisting of bronchial asthma, atopic dermatitis, allergic rhinitis, drug allergy, food allergy, anaphylaxis, allergic conjunctivitis, urticaria, eosinophilic sinusitis, eosinophilic gastrointestinal diseases and allergic bronchopulmonary aspergillosis.

6. A method of preventing and/or treating Th2-mediated diseases, comprising administering an effective amount of a PD-1 agonist to a subject.

7. A PD-1 agonist for use in preventing and/or treating Th2-mediated diseases.

8. Use of a PD-1 agonist for preventing and/or treating Th2-mediated diseases.

9. APD-1 agonist and use thereof for suppressing IgE production by suppression of Th2 type cytokines.

10. APD-1 agonist and use thereof for suppressing activation of eosinophils by suppression of Th2 type cytokines.
